(19) Europäisches Patentamt
European Patent Office
Office européen des brevets

(11) **EP 4 378 479 A2**

(12) **EUROPEAN PATENT APPLICATION**

(43) Date of publication:
**05.06.2024 Bulletin 2024/23**

(21) Application number: **24158584.3**

(22) Date of filing: **18.01.2022**

(51) International Patent Classification (IPC):
***A61K 39/395*** (2006.01)

(52) Cooperative Patent Classification (CPC):
**C07K 16/18; A61K 39/39591; A61P 7/00;**
**A61P 7/02; A61P 37/02;** A61K 2039/505;
C07K 2317/526; C07K 2317/76

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **22.01.2021 US 202163140488 P**

(62) Document number(s) of the earlier application(s) in accordance with Art. 76 EPC:
**22704040.9 / 4 281 472**

(71) Applicant: **ALEXION PHARMACEUTICALS, INC.**
**Boston, MA 02210 (US)**

(72) Inventors:
• **CHEN, Gin-Fu**
**Boston, 02210 (US)**

• **KHAWAJA, Zeeshan**
**Boston, 02210 (US)**

(74) Representative: **J A Kemp LLP**
**80 Turnmill Street**
**London EC1M 5QU (GB)**

Remarks:
•The complete document including Reference Table(s) and the Sequence Listing(s) can be downloaded from the EPO website
•This application was filed on 20.02.2024 as a divisional application to the application mentioned under INID code 62.
•Claims filed after the date of filing of the application / after the date of receipt of the divisional application (Rule 68(4) EPC).

(54) **METHODS OF TREATING COMPLEMENT MEDIATED THROMBOTIC MICROANGIOPATHY USING AN ANTI-C5 ANTIBODY**

(57) Provided are methods for clinical treatment of complement-mediated TMA (CM-TMA) (e.g., CM-TMA associated with a trigger, such as autoimmune condition, an infection, a transplant, one or more drugs, or malignant hypertension), using an anti-C5 antibody, or antigen binding fragment thereof, such as ravulizumab (ULTOMIRIS®).

Note: Randomized participants receive a weight-based loading dose on Day 1, followed by weight-based maintenance dosing on Day 15 and then q8w. Weight-based dosing regimen is based on last recorded study visit body weight.
Abbreviations: BSC = best supportive care; q8w = every 8 weeks; TMA = thrombotic microangiopathy.

**FIG. 1**

EP 4 378 479 A2

## Description

### CROSS REFERENCE TO RELATED APPLICATIONS

This application claims priority to, and the benefit of, U.S. Provisional Application No. 63/140,488, filed on January 22, 2021, the entire contents which are incorporated herein by reference.

### SEQUENCE LISTING

The instant application contains a Sequence Listing which has been submitted electronically in ASCII format and is hereby incorporated by reference in its entirety. Said ASCII copy, created on December 30, 2021, is named 0639WO_SL.txt and is 58,805 bytes in size.

### BACKGROUND

Thrombotic microangiopathy (TMA) is a rare, life-threatening disease often caused by complement activation that results in endothelial damage. In some patients, complement-mediated TMA (CM-TMA) may result from a trigger that injures the endothelium, such as post-partum, malignant hypertension (sometimes termed hypertensive emergency), infection, transplant (solid organ or bone marrow), autoimmune disease, and certain drugs (see, *e.g.,* Aigner C, et al., Clin. Kidney J. 2019;12(3):333-337; Go RS, et al., Mayo Clin. Proc. 2016;91(9):1189-1211; Goodship THJ, et al., Kidney Int. 2017;91(3):539-551; and Park, et al., Blood Advances. 2018;2(16):2090-2094). Many patients with CM-TMA present in critical condition, require management in an intensive care unit, and often need dialysis. Once multiorgan dysfunction develops, patients have a poor prognosis (see, *e.g.,* Le Clech A, et al., Kidney Intl. 2019;95(6):1443-1452).

Two complement C5 inhibitors, ravulizumab (ULTOMIRIS®) and eculizumab (SOLIRIS®), are approved for the treatment of patients with aHUS. There are currently no approved therapies for the broader complement mediated TMA population, especially in the case of CM-TMA with a trigger (also referred to as "secondary TMA"). Treatment typically consists of corticosteroids and/or therapeutic plasma exchange (TPE) or plasma infusion. The underlying trigger may also be treated, along with other supportive measures (*e.g.,* transfusion, dialysis), as appropriate. While TPE/plasma infusion improve hematologic parameters in CM-TMA, neither corticosteroids nor TPE/plasma infusion address the underlying complement dysregulation and the TMA process is likely to persist (see, *e.g.,* Azoulay E, et al., Chest. 2017;152(2):424-434; Kavanagh D, et al., Br. Med. Bull. 2006;77-78(5-22):5-22; and Laurence, Clin. Adv. Hematol. Oncol. 2013;11(Suppl 15):4-15). Removal of the trigger and supportive care are sometimes sufficient to reverse TMA symptoms. However, in patients with severe renal manifestations, outcomes are not greatly improved after removing/treating the trigger and providing supportive care. Accordingly, it is an object of the present invention to provide improved methods for treating patients with CM-TMA, especially, for the treatment of patients with triggered CM-TMA.

### SUMMARY

Provided herein are compositions and methods for treatment of complement-mediated TMA, a rare and potentially fatal disease with limited treatment options. Particularly, the compositions and methods of the present disclosure address unmet medical needs for treating severe renal dysfunction of CM-TMA.

Considering the high unmet need for effective therapies for CM-TMA, the present disclosure relates to use of anti-C5 antibodies, such as ravulizumab, for clinically improving outcomes of patients with CM-TMA, while minimizing risks associated with therapy.

Provided herein are compositions and methods for treating complement-mediated TMA (CM-TMA) in a human patient, comprising administering to the patient an anti-C5 antibody, or antigen binding fragment thereof (*e.g.,* ravulizumab (ULTOMIRIS®)). In some embodiments, the anti-C5 antibody, or antigen binding fragment thereof, is administered (or is for administration) according to a particular clinical dosage regimen (*i.e.*, at a particular dose amount and according to a specific dosing schedule). In some embodiments, the CM-TMA is associated with a trigger (also referred to as "secondary TMA"), such as an autoimmune condition, an infection, a transplant, one or more drugs, or malignant hypertension.

Any suitable anti-C5 antibody, or antigen binding fragment thereof, can be used in the methods described herein. An exemplary anti-C5 antibody is ravulizumab (also known as ULTOMIRIS®, ALXN1210 and antibody BNJ441) comprising the heavy and light chains having the sequences shown in SEQ ID NOs:14 and 11, respectively, or antigen binding fragments and variants thereof. In other embodiments, the antibody comprises the heavy and light chain complementarity determining regions (CDRs) or variable regions (VRs) of ravulizumab (ULTOMIRIS®). Accordingly, in one embodiment, the antibody comprises the CDR1, CDR2, and CDR3 domains of the heavy chain variable (VH) region of ravulizumab (ULTOMIRIS®) having the sequence shown in SEQ ID NO:12, and the CDR1, CDR2 and CDR3 domains of the light chain variable (VL) region of ravulizumab (ULTOMIRIS®) having the sequence shown in SEQ ID NO:8. In another embodiment, the antibody comprises CDR1, CDR2 and CDR3 heavy chain sequences as set forth in SEQ ID NOs:19, 18, and 3, respectively, and CDR1, CDR2 and CDR3 light chain sequences as set forth in SEQ ID NOs:4, 5, and 6, respectively. In another embodiment, the antibody comprises VH and VL regions having the amino acid sequences set forth in SEQ ID NO: 12 and SEQ ID NO:8, respectively. In another embodiment, the antibody comprises a heavy chain constant region as set forth in SEQ ID NO: 13.

In another embodiment, the antibody comprises a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434 of a native human IgG Fc constant region, each in EU numbering.

In another embodiment, the antibody comprises CDR1, CDR2 and CDR3 heavy chain sequences as set forth in SEQ ID NOs:19, 18, and 3, respectively, and CDR1, CDR2 and CDR3 light chain sequences as set forth in SEQ ID NOs:4, 5, and 6, respectively and a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434 of a native human IgG Fc constant region, each in EU numbering.

In another embodiment, the antibody binds to human C5 at pH 7.4 and 25°C with an affinity dissociation constant ($K_D$) that is in the range 0.1 nM $\leq K_D \leq$ 1 nM. In another embodiment, the antibody binds to human C5 at pH 6.0 and 25°C with a $K_D \geq$ 10 nM. In yet another embodiment, the [($K_D$ of the antibody or antigen-binding fragment thereof for human C5 at pH 6.0 and at 25°C)/($K_D$ of the antibody or antigen-binding fragment thereof for human C5 at pH 7.4 and at 25°C)] of the antibody is greater than 25.

Another exemplary anti-C5 antibody is described in US Patent Nos. 8,241,628 and 8,883,158. In one embodiment, the antibody, or antigen binding fragment thereof, comprises heavy chain CDR1, CDR2 and CDR3 domains having the sequences set forth in SEQ ID NOs: 21, 22, and 23, respectively, and light chain CDR1, CDR2 and CDR3 domains having the sequences set forth in SEQ ID NOs: 24, 25, and 26, respectively. In another embodiment, the antibody, or antigen binding fragment thereof, comprises the VH region having the sequence set forth in SEQ ID NO:27, and the VL region having the sequence set forth in SEQ ID NO:28.

Another exemplary anti-C5 antibody is also described in US Patent Nos. 8,241,628 and 8,883,158. In one embodiment, the antibody, or antigen binding fragment thereof, comprises heavy chain CDR1, CDR2 and CDR3 domains having the sequences set forth in SEQ ID NOs: 29, 30, and 31, respectively, and light chain CDR1, CDR2 and CDR3 domains having the sequences set forth in SEQ ID NOs: 32, 33, and 34, respectively. In another embodiment, the antibody comprises the VH region having the sequence set forth in SEQ ID NO: 35, and the VL region having the sequence set forth in SEQ ID NO: 36.

Another exemplary anti-C5 antibody is described in US2016/0176954A1. In one embodiment, the antibody, or antigen binding fragment thereof, comprises heavy chain CDR1, CDR2 and CDR3 domains having the sequences set forth in SEQ ID NOs: 37, 38, and 39, respectively, and light chain CDR1, CDR2 and CDR3 domains having the sequences set forth in SEQ ID NOs: 40, 41, and 42, respectively. In another embodiment, the antibody comprises the VH region having the sequence set forth in SEQ ID NO: 43, and the VL region having the sequence set forth in SEQ ID NO: 44.

Another exemplary anti-C5 antibody is described in Fukuzawa T. et al. (Sci. Rep. 7:1080, 2017). In another embodiment, the antibody, or antigen binding fragment thereof, comprises a heavy chain comprising SEQ ID NO: 45 and a light chain comprising SEQ ID NO: 46.

Another exemplary anti-C5 antibody is described in US20170355757. In one embodiment, the antibody comprises a heavy chain variable region comprising SEQ ID NO:47 and a light chain variable region comprising SEQ ID NO:48. In another embodiment, the antibody comprises a heavy chain comprising SEQ ID NO:49 and a light chain comprising SEQ ID NO:50.

In another embodiment, the antibody competes for binding with, and/or binds to the same epitope on C5 as, the above-mentioned antibodies. In another embodiment, the antibody has at least about 90% variable region amino acid sequence identity with the above-mentioned antibodies (*e.g.,* at least about 90%, 91%, 92%, 93%, 94%, 95%, 96%, 97%, 98% or 99% variable region identity).

In one embodiment, a method of treating a human patient with CM-TMA (e.g., CM-TMA associated with a trigger, such as an autoimmune condition, an infection, a transplant, one or more drugs, or malignant hypertension) is provided, the method comprising administering to the patient an effective amount of an anti-C5 antibody, or antigen binding fragment thereof, wherein the anti-C5 antibody, or antigen binding fragment thereof comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively. In another embodiment, the antibody further comprises a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434 of a native human IgG Fc constant region, each in EU numbering. In another embodiment, the anti-C5 antibody, or antigen binding fragment thereof, comprises a heavy chain variable region as set forth in SEQ ID NO:12 and a light chain variable region as set forth in SEQ ID NO:8. In another embodiment, the anti-C5 antibody, or antigen-binding fragment thereof, further comprises a heavy chain constant region depicted in SEQ ID NO:13. In another embodiment, the anti-C5 antibody, or antigen binding fragment thereof, comprises a heavy chain as set forth in SEQ ID NO:14 and a light chain as set forth in SEQ ID NO:11.

In one embodiment, the anti-C5 antibody, or antigen binding fragment is administered at a fixed dose. For example, in one embodiment, the anti-C5 antibody, or antigen binding fragment is administered at a dose of 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg, or 11000 mg, without regard to the patient's weight.

In another embodiment, the anti-C5 antibody, or antigen binding fragment thereof, (e.g., ravulizumab (ULTOMIRIS®)) is administered at a dose of 1200 mg, 2400 mg, 2700 mg, 3000 mg, 3300 mg, or 3600 mg.

In another embodiment, the dose of the anti-C5 antibody, or antigen binding fragment is based on the weight of the patient. For example, in one embodiment, 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg, or 11000 mg of the anti-C5 antibody, or antigen binding fragment is administered to a patient weighing ≥ 30 to < 40 kg. In another embodiment, 1200 mg or 2700 mg of the anti-C5 antibody, or antigen binding fragment thereof, (e.g., ravulizumab (ULTOMIRIS®)) is administered to a patient weighing ≥ 30 to < 40 kg.

In another embodiment, 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg, or 11000 mg of the anti-C5 antibody, or antigen binding fragment is administered to a patient weighing ≥ 40 to < 60 kg. In another embodiment, 2400 mg or 3000 mg of the anti-C5 antibody, or antigen binding fragment thereof, (e.g., ravulizumab (ULTOMIRIS®)) is administered to a patient weighing ≥ 40 to < 60 kg.

In another embodiment, 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg, or 11000 mg of the anti-C5 antibody, or antigen binding fragment thereof, is administered to a patient weighing ≥ 60 to < 100 kg. In another embodiment, 2700 mg or 3300 mg of the anti-C5 antibody, or antigen binding fragment thereof, (*e.g.,* ravulizumab (ULTOMIRIS®)) is administered to a patient weighing ≥ 60 to < 100 kg.

In another embodiment, 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg, or 11000 mg of the anti-C5 antibody, or antigen binding fragment, is administered to a patient weighing > 100 kg. In another embodiment, 3000 mg or 3600 mg of the anti-C5 antibody, or antigen binding fragment thereof, (*e.g.,* ravulizumab (ULTOMIRIS®)) is administered to a patient weighing ≥ 100 kg.

In another embodiment, a method of treating a human patient with CM-TMA (*e.g.,* CM-TMA associated with a trigger, such as an autoimmune condition, an infection, a transplant, one or more drugs, or malignant hypertension) is provided, the method comprising administering to the patient an anti-C5 antibody, or antigen binding fragment thereof (*e.g.,* ravulizumab (ULTOMIRIS®):

(a) once on Day 1 at a dose of: 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing > 100 kg; and
(b) on Day 15 and every eight weeks thereafter at a dose of 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

In another embodiment, a method of treating a human patient with CM-TMA (e.g., CM-TMA associated with a trigger, such as an autoimmune condition, an infection, a transplant, one or more drugs, or malignant hypertension) is provided, wherein the anti-C5 antibody, or antigen binding fragment thereof, (*e.g.,* ravulizumab (ULTOMIRIS®)) is administered to a patient weighing ≥ 30 to < 40 kg:

(a) once on Day 1 at a dose of 1200 mg; and
(b) on Day 15 and every eight weeks thereafter at a dose of 2700 mg.

In another embodiment, a method of treating a human patient with CM-TMA (*e.g.,* CM-TMA associated with a trigger, such as an autoimmune condition, an infection, a transplant, one or more drugs, or malignant hypertension) is provided, wherein the anti-C5 antibody, or antigen binding fragment thereof, (*e.g.,* ravulizumab (ULTOMIRIS®)) is administered to a patient weighing ≥ 40 to < 60 kg:

(a) once on Day 1 at a dose of 2400 mg; and
(b) on Day 15 and every eight weeks thereafter at a dose of 3000 mg.

In another embodiment, a method of treating a human patient with CM-TMA (*e.g.,* CM-TMA associated with a trigger, such as an autoimmune condition, an infection, a transplant, one or more drugs, or malignant hypertension) is provided, wherein the anti-C5 antibody, or antigen binding fragment thereof, (*e.g.,* ravulizumab (ULTOMIRIS®)) is administered to a patient weighing ≥ 60 to < 100 kg:

(a) once on Day 1 at a dose of 2700 mg; and

(b) on Day 15 of the administration cycle and every eight weeks thereafter at a dose of 3300 mg.

In another embodiment, a method of treating a human patient with CM-TMA (*e.g.,* CM-TMA associated with a trigger, such as an autoimmune condition, an infection, a transplant, one or more drugs, or malignant hypertension) is provided, wherein the anti-C5 antibody, or antigen binding fragment thereof, (*e.g.,* ravulizumab (ULTOMIRIS®)) is administered to a patient weighing ≥ 100 kg:

(a) once on Day 1 at a dose of 3000 mg; and
(b) on Day 15 and every eight weeks thereafter at a dose of 3600 mg.

In another embodiment, a method of treating a human patient with CM-TMA (*e.g.,* CM-TMA associated with a trigger, such as an autoimmune condition, an infection, a transplant, one or more drugs, or malignant hypertension) is provided, the method comprising administering to the patient an effective amount of an anti-C5 antibody, or antigen binding fragment thereof, wherein the anti-C5 antibody, or antigen binding fragment thereof, comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively, and a variant human Fc region that binds to human neonatal Fc receptor (FcRn), wherein the variant human Fc CH3 region comprises Met-429-Leu and Asn-435-Ser substitutions at residues corresponding to methionine 428 and asparagine 434 of a native human IgG Fc region, each in EU numbering, and wherein the anti-C5 antibody, or antigen binding fragment thereof, is administered to the patient

(a) once on Day 1 at a dose of: 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing > 100 kg; and
(b) on Day 15 and every eight weeks thereafter at a dose of 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

In another embodiment, the anti-C5 antibody, or antigen binding fragment, is administered at a milligram per kilogram (mg/kg) dose. For example, in one embodiment, the anti-C5 antibody, or antigen binding fragment thereof, is administered at a dose of 0.1 mg/kg, 0.25 mg/kg, 0.5 mg/kg, 0.75 mg/kg, 1.0 mg/kg, 1.25 mg/kg, 1.50 mg/kg, 1.75 mg/kg, 2.0 mg/kg, 2.25 mg/kg, 2.50 mg/kg, 2.75 mg/kg, 3.0 mg/kg, 3.25 mg/kg, 3.50 mg/kg, 3.75 mg/kg, 4.0 mg/kg, 4.25 mg/kg, 4.50 mg/kg, 4.75 mg/kg, 5.0 mg/kg, 5.25 mg/kg, 5.50 mg/kg, 5.75 mg/kg, 6.0 mg/kg, 6.25 mg/kg, 6.50 mg/kg, 6.75 mg/kg, 7.0 mg/kg, 7.25 mg/kg, 7.50 mg/kg, 7.75 mg/kg, 8.0 mg/kg, 8.25 mg/kg, 8.50 mg/kg, 8.75 mg/kg, 9.0 mg/kg, 9.25 mg/kg, 9.50 mg/kg, 9.75 mg/kg, 10.0 mg/kg, 11.25 mg/kg, 11.50 mg/kg, 11.75 mg/kg, 12.0 mg/kg, 12.25 mg/kg, 12.50 mg/kg, 12.75 mg/kg, 13.0 mg/kg, 13.25 mg/kg, 13.50 mg/kg, 13.75 mg/kg, 14.0 mg/kg, 14.25 mg/kg, 14.50 mg/kg, 14.75 mg/kg, 15.0 mg/kg, 15.25 mg/kg, 15.50 mg/kg, 15.75 mg/kg, 16.0 mg/kg, 16.25 mg/kg, 16.50 mg/kg, 16.75 mg/kg, 17.0 mg/kg, 17.25 mg/kg, 17.50 mg/kg, 17.75 mg/kg, 18.0 mg/kg, 18.25 mg/kg, 18.50 mg/kg, 18.75 mg/kg, 19.0 mg/kg, 19.25 mg/kg, 19.50 mg/kg, 19.75 mg/kg, 20.0 mg/kg, 20.25 mg/kg, 20.50 mg/kg, 20.75 mg/kg, 21.0 mg/kg, 21.25 mg/kg, 21.50 mg/kg, 21.75 mg/kg, 22.0 mg/kg, 22.25 mg/kg, 22.50 mg/kg, 22.75 mg/kg, 23.0 mg/kg, 23.25 mg/kg, 23.50 mg/kg, 23.75 mg/kg, 24.0 mg/kg, 24.25 mg/kg, 24.50 mg/kg, 24.75 mg/kg, or 25.0 mg/kg.

In one embodiment, the anti-C5 antibody, or antigen binding fragment is administered once per week, twice per week, three times per week, four times per week, five times per week, six times per week, or daily. In another embodiment, anti-C5 antibody, or antigen binding fragment, is administered once every two weeks, once every three weeks, once every four weeks, once every five weeks, once every six weeks, once every seven weeks, once every eight weeks, once every nine weeks, once every ten weeks, once every eleven weeks, or once every twelve weeks. In another embodiment, the anti-C5 antibody, or antigen binding fragment, is administered at a loading dose on Day 1, followed by a different maintenance dose on Day 15 and every eight weeks thereafter.

In another embodiment, the anti-C5 antibody, or antigen binding fragment thereof, is administered for one or more administration cycles. In one embodiment, the administration cycle is 26 weeks. In another embodiment, the treatment comprises at least 1, 2, 3, 4, 5, 6, 7, 8, 9, 10, or 11 cycles. In another embodiment, the patient is treated for about 1, 2, 3, 4, 5, or 6 months. In another embodiment, the treatment continues for the lifetime of the human patient.

The anti-C5 antibody, or antigen binding fragment, can be administered via any suitable means. In one embodiment, the anti-C5 antibody, or antigen binding fragment (e.g., ravulizumab (ULTOMIRIS®)), is administered intravenously. In another embodiment, the anti-C5 antibody, or antigen binding fragment, is administered subcutaneously.

In some embodiments, the patients treated according to the methods described herein have been vaccinated against meningococcal infections within 3 years prior to, or at the time of, initiating treatment. In one embodiment, patients who received treatment less than 2 weeks after receiving a meningococcal vaccine are also treated with appropriate prophylactic antibiotics until 2 weeks after vaccination. In another embodiment, patients treated according to the methods described herein are vaccinated against meningococcal serotypes A, C, Y, W135, and/or B.

In some embodiments, the patients treated according to the methods have TMA associated with lupus nephritis, systemic sclerosis, or solid organ transplant. In one embodiment, these patients are vaccinated against Haemophilus influenzae type b (Hib) and Streptococcus pneumoniae prior to treatment.

In another aspect, the treatment regimens described are sufficient to maintain particular serum trough concentrations of the anti-C5 antibody, or antigen binding fragment thereof. For example, in one embodiment, the treatment can maintain a serum trough concentration of the anti-C5 antibody, or antigen binding fragment thereof, of 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, 205, 210, 215, 220, 225, 230, 240, 245, 250, 255, 260, 265, 270, 280, 290, 300, 305, 310, 315, 320, 325, 330, 335, 340, 345, 350, 355, 360, 365, 370, 375, 380, 385, 390, 395, or 400 $\mu$g/mL or greater. In one embodiment, the treatment maintains a serum trough concentration of the anti-C5 antibody, or antigen binding fragment thereof, of 100 $\mu$g/mL or greater. In another embodiment, the treatment maintains a serum trough concentration of the anti-C5 antibody, or antigen binding fragment thereof, of 150 $\mu$g/mL or greater. In another embodiment, the treatment maintains a serum trough concentration of the anti-C5 antibody, or antigen binding fragment thereof, of 200 $\mu$g/mL or greater. In another embodiment, the treatment maintains a serum trough concentration of the anti-C5 antibody, or antigen binding fragment thereof, of 250 $\mu$g/mL or greater. In another embodiment, the treatment maintains a serum trough concentration of the anti-C5 antibody, or antigen binding fragment thereof, of 300 $\mu$g/mL or greater. In another embodiment, the treatment maintains a serum trough concentration of the anti-C5 antibody, or antigen binding fragment thereof, of between 100 $\mu$g/ml and 200 $\mu$g/mL. In another embodiment, the treatment maintains a serum trough concentration of the anti-C5 antibody, or antigen binding fragment thereof, of about 175 $\mu$g/mL.

In another embodiment, to obtain an effective response, the anti-C5 antibody is administered to the patient in an amount and with a frequency to maintain at least 50 $\mu$g, 55$\mu$g, 60 $\mu$g, 65 $\mu$g, 70 $\mu$g, 75 $\mu$g, 80 $\mu$g, 85 $\mu$g, 90 $\mu$g, 95 $\mu$g, 100 $\mu$g, 105 $\mu$g, 110 $\mu$g, 115 $\mu$g, 120 $\mu$g, 125 $\mu$g, 130 $\mu$g, 135 $\mu$g, 140 $\mu$g, 145 $\mu$g, 150 $\mu$g, 155 $\mu$g, 160 $\mu$g, 165 $\mu$g, 170 $\mu$g, 175 $\mu$g, 180 $\mu$g, 185 $\mu$g, 190 $\mu$g, 195 $\mu$g, 200 $\mu$g, 205 $\mu$g, 210 $\mu$g, 215 $\mu$g, 220 $\mu$g, 225 $\mu$g, 230 $\mu$g, 235 $\mu$g, 240 $\mu$g, 245 $\mu$g, 250 $\mu$g, 255 $\mu$g, or 260 $\mu$g of antibody per milliliter of the patient's blood. In another embodiment, the anti-C5 antibody is administered to the patient in an amount and with a frequency to maintain between 50 $\mu$g and 250 $\mu$g of antibody per milliliter of the patient's blood. In another embodiment, the anti-C5 antibody is administered to the patient in an amount and with a frequency to maintain between 100 $\mu$g and 200 $\mu$g of antibody per milliliter of the patient's blood. In another embodiment, the anti-C5 antibody is administered to the patient in an amount and with a frequency to maintain about 175 $\mu$g of antibody per milliliter of the patient's blood.

In another embodiment, to obtain an effective response, the anti-C5 antibody is administered to the patient in an amount and with a frequency to maintain a minimum free C5 concentration. For example, in one embodiment, the anti-C5 antibody can be administered to the patient in an amount and with a frequency to maintain a free C5 concentration of 0.2 $\mu$g/mL, 0.3 $\mu$g/mL, 0.4 $\mu$g/mL, 0.5 $\mu$g/mL or below. In another embodiment, the treatment described herein reduces free C5 concentration by greater than 99% throughout the treatment period.

In one aspect of the invention, the CM-TMA treated according to the methods described herein is associated with a trigger. In one embodiment, the CM-TMA trigger is an autoimmune condition or event. Exemplary autoimmune conditions include, but are not limited to: acquired aplastic anemia, acute disseminated encephalomyelitis (ADEM), acute hemorrhagic leukoencephalitis (AHLE) / Hurst's disease, gammaglobulinemia, (primary), alopecia areata, ankylosing spondylitis (AS), anti-NMDA receptor encephalitis, antiphospholipid syndrome (APS), arteriosclerosis, autism spectrum disorders (ASD), autoimmune Addison's disease (AAD), autoimmune dysautonomia / Autoimmune autonomic ganglionopathy (AAG), autoimmune encephalitis, autoimmune gastritis, autoimmune hemolytic anemia (AIHA), autoimmune hepatitis (AIH), autoimmune hyperlipidemia, autoimmune hypophysitis / lymphocytic hypophysitis, autoimmune inner ear disease (AIED), autoimmune lymphoproliferative syndrome (ALPS), autoimmune myocarditis, autoimmune oophoritis, autoimmune orchitis, autoimmune pancreatitis (AIP) / Immunoglobulin G4-Related Disease (IgG4-RD), autoimmune polyglandular syndromes (Types I, II, & III), autoimmune progesterone dermatitis, autoimmune sudden sensorineural hearing loss (SNHL), balo disease, behçet's disease, birdshot chorioretinopathy / birdshot uveitis, bullous pemphigoid, castleman disease, celiac disease, chagas disease, chronic fatigue syndrome (CFS) / myalgic encephalomyelitis (ME), chronic inflammatory demyelinating polyneuropathy (CIDP), chronic Lyme disease / post-treatment Lyme disease syndrome (PTLDS), chronic urticaria (CU), churg-Strauss syndrome / eosinophilic granulomatosis with polyangiitis (EGPA), cicatricial pemphigoid, cogan's syndrome, cold agglutinin disease, CREST syndrome / limited cutaneous systemic sclerosis, crohn's disease (CD), cronkhite-Canada syndrome (CSS), cryptogenic organizing pneumonia (COP), dermatitis herpetiformis, dermatomyositis, type 1 diabetes, discoid lupus, eressler's syndrome / postmyocardial infarction / postpericardiotomy syndrome, endometriosis, eosinophilic fasciitis, erythema nodosum, essential mixed cryoglobulinemia, evans syndrome, fibrosing alveolitis, giant cell arteritis / temporal arteritis / Horton's disease, glomerulonephritis, goodpasture's syndrome / anti-GBM/anti-TBM disease, granulomatosis with polyangiitis (GPA) / Wegener's granulomatosis, grave's disease, guillain-barré syndrome (GBS), hashimoto's thyroiditis / chronic lymphocytic thyroiditis / autoimmune thyroiditis, henoch-Schönlein purpura / IgA vasculitis, herpes gestationis / pemphigoid gestationis, hypogammaglobulinemia, IgA nephropathy / Berger's disease, immune thrombocytopenia (ITP) / autoimmune thrombocytopenic purpura, interstitial cystitis, juvenile idiopathic arthritis, kawasaki disease, lambert-eaton myasthenic syndrome (LEMS), leukocytoclastic vasculitis, lichen planus, lichen sclerosus, ligneous conjunctivitis, linear IgA disease (LAD) / linear IgA bullous dermatosis (LABD), lupus nephritis, ménière's disease, microscopic polyangiitis (MPA), mixed connective tissue disease (MCTD), mooren's ulcer, mucha-Habermann disease, multiple sclerosis (MS), myasthenia gravis (MG), neuromyelitis optica (NMO) / Devic's disease, narcolepsy, non-length-dependent small fiber sensory neuropathy (SFSN), ocular cicatricial pemphigoid, opsoclonus-myoclonus syndrome (OMS), palindromic rheumatism, palmoplantar pustulosis (type of psoriasis), paraneoplastic cerebellar degeneration, paraneoplastic pemphigus, paroxysmal nocturnal hemoglobinuria (PNH), peripheral uveitis / pars planitis, parsonage turner syndrome, pemphigus foliaceus, pemphigus vulgaris, pernicious anemia, POEMS syndrome, polyarteritis nodosa, polymyalgia rheumatica, polymyositis, postural orthostatic tachychardia syndrome (POTS), primary biliary cholangitis (PBC) / primary biliary cirrhosis, primary sclerosing cholangitis (PSC), psoriasis, psoriatic arthritis, pulmonary fibrosis, idiopathic (IPF), pure red cell aplasia, pyoderma gangrenosum, raynaud's phenomenon, reactive arthritis / Reiter's syndrome, reflex sympathetic dystrophy syndrome, relapsing polychondritis, leg syndrome / Willis-Ekbom disease, rheumatic fever, rheumatoid arthritis, sarcoidosis, schmidt syndrome / autoimmune poly endocrine syndrome type II, systemic sclerosis, scleritis, scleroderma, serpiginous choroidopathy, sjögren's syndrome, stiff person syndrome (SPS), systemic lupus erythematosus (SLE), subacute bacterial endocarditis (SBE), sydenham's chorea, sympathetic ophthalmia, takayasu's arteritis (vasculitis), testicular autoimmunity (vasculitis / orchitis), tolosa-Hunt syndrome, transverse myelitis (TM), tubulointerstitial nephritis uveitis syndrome (TINU), ulcerative colitis (UC), undifferentiated connective tissue disease (UCTD), uveitis (anterior), uveitis (intermediate), uveitis (posterior), vasculitis, vitiligo, and vogt-Koyanagi-Harada syndrome (VKH). In one embodiment, the autoimmune trigger is lupus nephritis. In another embodiment, the autoimmune trigger is systemic sclerosis.

In another embodiment, the CM-TMA triggered is an infection, such as a bacterial infection, viral infection, fungal infection, or parasitic infection. In one embodiment, the trigger is bacterial infection selected from the group consisting of strep throat, abacterial urinary tract infection (UTIs) (*e.g.,* often caused by coliform bacteria), bacterial food poisoning (*e.g.,* often caused by E. coli, Salmonella, or Shigella), bacterial cellulitis (*e.g.,* such as due to Staphylococcus aureus (MRSA),bacterial vaginosis, gonorrhea, chlamydia, syphilis, Clostridium difficile (C. diff), tuberculosis, whooping cough, pneumococcal pneumonia, bacterial meningitis, Lyme disease cholera, botulism, tetanus, and anthrax.

In another embodiment, the trigger is a viral infection selected from the group consisting of influenza (the flu), the common cold, measles, rubella, chickenpox, norovirus, polio, infectious mononucleosis (mono), herpes simplex virus (HSV), human papillomavirus (HPV) human immunodeficiency virus (HIV), viral hepatitis, which can include hepatitis A, B, C, D, and E, viral meningitis, West Nile Virus, rabies, ebola, and COVID-19.

In another embodiment, the trigger is a fungal infection selected from the group consisting of a yeast infection, ringworm, athlete's foot, thrush, aspergillosis, histoplasmosis. cryptococcus infection, and fungal meningitis.

In another embodiment, the trigger is a parasitic infection selected from the group consisting of malaria, toxoplasmosis, trichomoniasis, giardiasis, tapeworm infection, roundworm infection, lice, scabies, leishmaniasis, and river blindness.

In another embodiment, the trigger is not due to Shiga toxin-producing *Escherichia coli*, *e.g.*, Shiga toxin-related hemolytic uremic syndrome (STEC-HUS).

In another embodiment, the CM-TMA trigger is a transplant. In one embodiment, the trigger is a bone marrow transplant. In another embodiment, the trigger is a solid organ transplant (e.g., selected from the group consisting of a kidney, pancreas, liver, heart, and small bowel transplant).

In another embodiment, the CM-TMA trigger is one or more drugs. In some embodiments, the TMA is triggered by a drug with an immune-mediated mode of action (*e.g.,* quinine). In some embodiments, the TMA is triggered by a drug with a toxic mode of action (*e.g.,* cyclosporine or tacrolimus). In specific embodiments, the TMA is triggered by a drug selected from clopidogrel, cyclosporine, estrogen/progesterone, gemcitabine, interferons, mitomycin, quinine, tacrolimus, ticlopidine, or a combination thereof.

In another embodiment, the CM-TMA trigger is malignant hypertension. Malignant hypertension is extremely high blood pressure (*e.g.,* above 180/120) that develops rapidly and causes some type of organ damage.

In another aspect, the methods of treating with CM-TMA (*e.g.,* CM-TMA associated with a trigger, such as an autoimmune condition, an infection, a transplant, one or more drugs, or malignant hypertension) described herein can be used alone or in combination with one more additional therapies and/or therapeutic agents. For example, in one embodiment, the method further comprises administering to the patient best supportive care. Best supportive care includes, but is not limited to, (a) transfusion support, (b) anti-infectives (e.g., antibiotics, antivirals, and antifungals), (c) renal replacement therapy (dialysis), (d) antihypertensive medications, (e) therapy for TMA associated with lupus nephritis or SSc-TMA, and/or (f) withdrawal or dose adjustment of the suspected agent for drug-induced TMA.

The efficacy of the treatment methods provided herein can be assessed using any suitable means. In some embodiments, the treatment results in terminal complement inhibition.

In other embodiments, the treatment results in a normalization of platelet count without transfusion support and normalization of LDH levels.

In other embodiments, the treatment results in an improvement of eGFR of ≥ 30% compared to baseline. In other embodiments, the treatment results in a normalization of platelet count without transfusion support, normalization of LDH levels, and an improvement of eGFR of ≥ 30% compared to baseline. In other embodiments, the treatment results in a complete TMA response.

In other embodiments, the treatment results in LDH normalization (≤246 U/L). In other embodiments, the treatment results in LDH normalization (≤246 U/L) for at least 28 days (*e.g.,* at least 28 days, 1 month, 2 months, 3 months, 4 months, 5 months, 6 months, 7 months, 8 months, 9 months, 10 months, 11 months, 1 year, or two years). In other embodiments, the treatment results in LDH normalization 5-12 days after initiating treatment. For example, in one embodiment, LDH normalization occurs 5, 6, 7, 8, 9, 10, 11, or 12 days after initiating treatment.

In other embodiments, the treatment results in a complete TMA response for at least 28 days (*e.g.,* at least 28 days, 1 month, 2 months, 3 months, 4 months, 5 months, 6 months, 7 months, 8 months, 9 months, 10 months, 11 months, 1 year, or two years). In other embodiments, the treatment confers complete TMA response in the patient in fewer than about 60 days (e.g., 60, 59, 58, 57 56, 55, 54, 53, 52, 51, 50, 49, 48, 48, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, or 1 day(s)).

In other embodiments, the treatment results in hematological normalization.

In other embodiments, the treatment produces a reduction in the need for blood transfusions. In another embodiment, the treatment produces a greater than 70% increase in transfusion avoidance.

In other embodiments, the treatment produces a shift toward normal levels of one or more biomarkers selected from the group consisting of sTNF-RI, thrombomodulin, sVCAM-1, sC5b-9, C5a, factor Ba, and/or neutrophil gelatinase-associated lipocalin (NGAL).

In other embodiments, the treatment produces a change from baseline in quality of life, as assessed via the Functional Assessment of Chronic Illness Therapy (FACIT)-Fatigue Scale, the EuroQol 5-Dimension 5-Level (EQ-5D-5L) Scale, or the Kidney Disease Quality of Life instrument (KDQOL-36) Scale.

In other embodiments, the treatment prolongs survival of the patient.

In some embodiments, the patient with CM-TMA is not (a) a patient with aHUS, including postpartum aHUS (p-aHUS), or any known gene mutation that causes aHUS; (b) a patient with chronic kidney disease (CKD); (c) a patient who has developed TMA due to hematopoietic stem cell transplantation (HSCT-TMA); (d) a patient with a history of primary and secondary glomerular diseases other than lupus; (e) a patient with primary antiphospholipid antibody syndrome (APS); (f) a patient with history of Shiga toxin-producing Escherichia coli infection, e.g., Shiga toxin-related hemolytic uremic syndrome (STEC-HUS); (g) a patient with familial or acquired ADAMTS13 (a disintegrin and metalloproteinase with a thrombospondin type 1 motif, member 13) deficiency, e.g., wherein the ADAMTS13 deficiency is attributed to ADAMTS13 activity of < 5%; (h) a patient who is positive on a direct Coombs test; (i) a patient whose kidney biopsy is positive for interstitial fibrosis tubular atrophy, glomerulosclerosis, or crescent formation of at least 50%; or (j) a transplant patient with evidence of cellular or antibody-mediated graft rejection (AMR); or (k) a patient with any combination of features provided under (a)-(j). Particularly, the patient with CM-TMA is not a patient with aHUS (including p-aHUS).

Further provided are kits for treating CM-TMA (*e.g.,* CM-TMA associated with a trigger, such as an autoimmune condition, an infection, a transplant, one or more drugs, or malignant hypertension). In one embodiment, the kit comprises (a) a dose of an anti-C5 antibody, or antigen binding fragment thereof (*e.g.,* any of those previously described herein); and (b) instructions for using the anti-C5 antibody, or antigen binding fragment thereof, in the methods described herein.

In another embodiment, a kit for treating CM-TMA (*e.g.,* CM-TMA associated with a trigger, such as an autoimmune condition, an infection, a transplant, one or more drugs, or malignant hypertension) in a human patient is provided, the kit comprising: (a) a dose of an anti-C5 antibody, or antigen binding fragment thereof, wherein the anti-C5 antibody, or antigen binding fragment thereof, comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively; and (b) instructions for using the anti-C5 antibody, or antigen binding fragment thereof, in the methods described herein.

In another embodiment, a kit for treating CM-TMA (*e.g.,* CM-TMA associated with a trigger, such as an autoimmune condition, an infection, a transplant, one or more drugs, or malignant hypertension) in a human patient is provided, the kit comprising: (a) a dose of an anti-C5 antibody, or antigen binding fragment thereof, wherein the anti-C5 antibody, or antigen binding fragment thereof, comprises CDR1, CDR2 and CDR3 heavy chain sequences as set forth in SEQ ID NOs: 19, 18 and 3, respectively, and CDR1, CDR2 and CDR3 light chain sequences as set forth in SEQ ID NOs:4, 5 and 6, respectively, and a variant human Fc region that binds to human neonatal Fc receptor (FcRn), wherein the variant human Fc CH3 region comprises Met-429-Leu and Asn-435-Ser substitutions at residues corresponding to methionine 428 and asparagine 434 of a native human IgG Fc region, each in EU numbering; and (b) instructions for using the anti-C5 antibody, or antigen binding fragment thereof, in the methods described herein.

In another embodiment, a kit for treating CM-TMA (*e.g.,* CM-TMA associated with a trigger, such as an autoimmune condition, an infection, a transplant, one or more drugs, or malignant hypertension) in a human patient is provided, the kit comprising: (a) a dose of ravulizumab (ULTOMIRIS®) and (b) instructions for using ravulizumab (ULTOMIRIS®) in the methods described herein. Particularly, in some embodiments, the kit for treating triggered CM-TMA as provided herein excludes kits for treating CM-TMA triggered by Shiga toxin-producing *Escherichia coli* infection, *e.g.,* Shiga toxin-related hemolytic uremic syndrome; and/or CM-TMA triggered by hematopoietic stem cell transplant (HSCT).

In some embodiments, the disclosure relates to use of a therapeutically effective amount of an anti-C5 antibody (*e.g.,* eculizumab or ravulizumab (preferably ravulizumab) for the treatment of complement-mediated TMA (CM-TMA), particularly, CM-TMA with a trigger, in a patient. Particularly, the disclosure relates to use of a therapeutically effective amount of an anti-C5 antibody (*e.g.,* eculizumab or ravulizumab (preferably ravulizumab) for the treatment of CM-TMA in a patient, wherein the CM-TMA is not due to atypical hemolytic uremic syndrome (aHUS) or postpartum aHUS (p-aHUS).

In some embodiments, the disclosure relates to a use, according to the foregoing, of a therapeutically effective amount of eculizumab or ravulizumab (preferably ravulizumab) in the treatment of CM-TMA triggered by at least one of: (a) an autoimmune condition (except AMR); (b) an infection (except STEC infection); (c) a transplant (except hematopoietic stem cell transplant (HSCT)); (d) one or more drugs; or (e) malignant hypertension, in a patient.

In some embodiments, the disclosure relates to a use, according to the foregoing, of a therapeutically effective amount of eculizumab or ravulizumab (preferably ravulizumab) ) in the treatment of CM-TMA in a patient, wherein the patient is not (a) a patient with aHUS, including postpartum aHUS, or any known gene mutation that causes aHUS; (b) a patient with chronic kidney disease (CKD); (c) a patient who has developed TMA due to hematopoietic stem cell transplantation (HSCT-TMA); (d) a patient with a history of primary and secondary glomerular diseases other than lupus; (e) a patient with primary antiphospholipid antibody syndrome (APS); (f) a patient with history of Shiga toxin-producing Escherichia coli infection, e.g., Shiga toxin-related hemolytic uremic syndrome (STEC-HUS); (g) a patient with familial or acquired ADAMTS13 (a disintegrin and metalloproteinase with a thrombospondin type 1 motif, member 13) deficiency, e.g., wherein the ADAMTS13 deficiency is attributed to ADAMTS13 activity of less than 5%; (h) a patient who is positive on a direct Coombs test; (i) a patient whose kidney biopsy is positive for interstitial fibrosis tubular atrophy, glomerulosclerosis, or crescent formation of at least 50%; (j) a transplant patient with evidence of cellular or antibody-mediated graft rejection (AMR); or (k) a patient with any combination of features provided under (a)-(j), above.

## BRIEF DESCRIPTION OF THE DRAWING

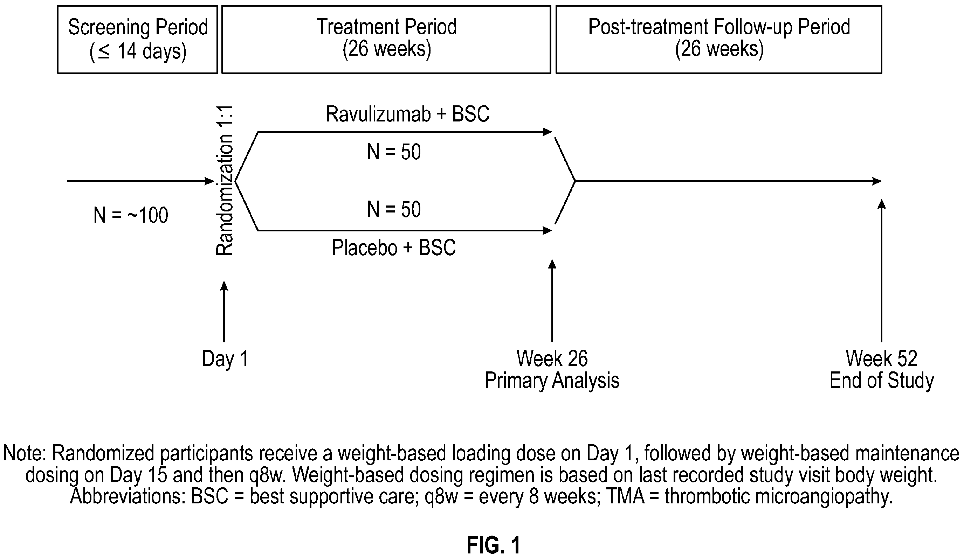

**FIG. 1** is a schematic depicting the study design.

## DETAILED DESCRIPTION

I. Anti-C5 Antibodies

The anti-C5 antibodies described herein bind to complement component C5 (*e.g.,* human C5) and inhibit the cleavage of C5 into fragments C5a and C5b. As described above, such antibodies also have, for example, improved pharmacokinetic properties relative to other anti-C5 antibodies (*e.g.,* eculizumab) used for therapeutic purposes.

The term "antibody" describes polypeptides comprising at least one antibody derived antigen binding site (*e.g.,* $V_H/V_L$ region or $F_v$, or CDR). Antibodies include known forms of antibodies. An antibody can be, for example, a human antibody, a humanized antibody, a bispecific antibody or a chimeric antibody. An antibody also can be a Fab, Fab'2, ScFv, SMIP, Affibody®, nanobody or a domain antibody. An antibody also can be of any of the following isotypes: IgG1, IgG2, IgG3, IgG4, IgM, IgA1, IgA2, IgAsec, IgD, IgE or a hybrid of any of these isotypes. An antibody can be a naturally occurring antibody or an antibody that has been altered by a protein engineering technique (*e.g.,* by mutation, deletion, substitution, conjugation to a non-antibody moiety). An antibody can include, for example, one or more variant amino acids (compared to a naturally occurring antibody), which changes a property (*e.g.,* a functional property) of the antibody. Numerous such alterations are known in the art that affect, *e.g.,* half-life, effector function, and/or immune responses to the antibody in a patient. The term "antibody" also includes artificial or engineered polypeptide constructs that comprise at least one antibody-derived antigen binding site.

Anti-C5 antibodies (or $V_H/V_L$ domains derived therefrom) suitable for use herein can be generated using methods known in the art. Alternatively, art-recognized anti-C5 antibodies can be used. Antibodies that compete with any of these art-recognized antibodies for binding to C5 also can be used.

Eculizumab (also known as SOLIRIS®) is an anti-C5 antibody comprising heavy chain CDR1, CDR2 and CDR3 domains having the sequences set forth in SEQ ID NOs:1, 2 and 3, respectively, and light chain CDR1, CDR2 and CDR3 domains having the sequences set forth in SEQ ID NOs:4, 5 and 6, respectively. Eculizumab comprises a heavy chain variable region having the amino acid sequence set forth in SEQ ID NO:7 and a light chain variable region having the amino acid sequence set forth in SEQ ID NO:8. The variable regions of eculizumab are described in PCT/US1995/005688 and US Patent No:6,355,245, the teachings of which are hereby incorporated by reference in their entirety. Eculizumab comprises a heavy chain comprising the amino acid sequence set forth in SEQ ID NO: 10 and a light chain having the amino acid sequence set forth in SEQ ID NO:11. The full heavy and light chains of eculizumab are described in PCT/US2007/006606, the entire teachings of which are hereby incorporated by reference. In some embodiments, eculizumab includes a biosimilar of SOLIRIS®. As used herein, a biosimilar is a product which is highly similar (*e.g.,* in structure, function and property) to another already approved biological medicine (*e.g.,* a reference medicine). Representative examples of SOLIRIS® biosimilars include, *e.g.,* monoclonal antibody ABP 959; ELIZARIA; and monoclonal antibody SB12.

An exemplary anti-C5 antibody is ravulizumab comprising heavy and light chains having the sequences shown in SEQ ID NOs:14 and 11, respectively, or antigen binding fragments and variants thereof. Ravulizumab (also known as ULTOMIRIS®) is described in PCT/US2015/019225 and US Patent No.: 9,079,949, the entire teachings of which are hereby incorporated by reference. Ravulizumab selectively binds to human complement protein C5, inhibiting its cleavage to C5a and C5b during complement activation. This inhibition prevents the release of the proinflammatory mediator C5a and the formation of the cytolytic pore-forming membrane attack complex (MAC) C5b-9 while preserving the proximal or early components of complement activation (*e.g.,* C3 and C3b) essential for the opsonization of microorganisms and clearance of immune complexes.

In other embodiments, the antibody comprises the heavy and light chain CDRs or variable regions of ravulizumab (ULTOMIRIS®). The antibody can comprise, for example, the CDR1, CDR2 and CDR3 domains of the $V_H$ region of ravulizumab (ULTOMIRIS®) having the sequence set forth in SEQ ID NO:12, and the CDR1, CDR2 and CDR3 domains of the $V_L$ region of ravulizumab (ULTOMIRIS®) having the sequence set forth in SEQ ID NO:8. In another embodiment, the antibody comprises heavy chain CDR1, CDR2 and CDR3 domains having the sequences set forth in SEQ ID NOs:19, 18 and 3, respectively, and light chain CDR1, CDR2 and CDR3 domains having the sequences set forth in SEQ ID NOs:4, 5 and 6, respectively. In another embodiment, the antibody comprises $V_H$ and $V_L$ regions having the amino acid sequences set forth in SEQ ID NO:12 and SEQ ID NO:8, respectively.

Another exemplary anti-C5 antibody comprises heavy and light chains having the sequences shown in SEQ ID NOs:20 and 11, respectively, or antigen binding fragments and variants thereof. In other embodiments, the antibody can comprise the heavy and light chain CDRs of SEQ ID Nos:20 and 11. Accordingly, in one embodiment, the antibody comprises the CDR1, CDR2 and CDR3 domains of the $V_H$ having the sequence set forth in SEQ ID NO:12, and the CDR1, CDR2 and CDR3 domains of the $V_L$ region having the sequence set forth in SEQ ID NO:8. In another embodiment, the antibody comprises heavy chain CDR1, CDR2 and CDR3 domains having the sequences set forth in SEQ ID NOs:19, 18 and 3, respectively, and light chain CDR1, CDR2 and CDR3 domains having the sequences set forth in SEQ ID NOs:4, 5 and 6, respectively.

The exact boundaries of CDRs have been defined differently according to different methods. In some embodiments, the positions of the CDRs or framework regions within a light or heavy chain variable domain can be as defined by Kabat et al. ("Sequences of Proteins of Immunological Interest." NIH Publication No. 91-3242, U.S. Department of Health and Human Services, Bethesda, MD, 1991). In such cases, the CDRs can be referred to as "Kabat CDRs" (*e.g.,* "Kabat LCDR2" or "Kabat HCDR1"). In some embodiments, the positions of the CDRs of a light or heavy chain variable region can be as defined by Chothia et al. (Nature, 342:877-83, 1989). Accordingly, these regions can be referred to as "Chothia CDRs" (*e.g.,* "Chothia LCDR2" or "Chothia HCDR3"). In some embodiments, the positions of the CDRs of the light and heavy chain variable regions can be as defined by a Kabat-Chothia combined definition. In such embodiments, these regions can be referred to as "combined Kabat-Chothia CDRs" (Thomas et al., Mol. Immunol., 33:1389-401, 1996).

In another embodiment, the antibody comprises $V_H$ and $V_L$ regions having the amino acid sequences set forth in SEQ ID NO:12 and SEQ ID NO:8, respectively. In another embodiment, the antibody comprises a heavy chain constant region as set forth in SEQ ID NO:13. In another embodiment, the antibody comprises a heavy chain polypeptide as set forth in SEQ ID NO:14 and a light chain polypeptide as set forth in SEQ ID NO:11. In another embodiment, the antibody comprises a variant human Fc region that binds to human neonatal Fc receptor (FcRn), wherein the variant human Fc CH3 region comprises Met429Leu and Asn435Ser substitutions at residues corresponding to methionine 428 and asparagine 434 of a native human IgG Fc region, each in EU numbering.

In another embodiment, the antibody comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively and a variant human Fc region that binds to human neonatal Fc receptor (FcRn), wherein the variant human Fc CH3 region comprises Met429Leu and Asn435Ser substitutions at residues corresponding to methionine 428 and asparagine 434 of a native human IgG Fc region, each in EU numbering.

In another embodiments, an anti-C5 antibody described herein comprises a heavy chain CDR1 comprising, or consisting of, the following amino acid sequence: GHIFSNYWIQ (SEQ ID NO:19). In another embodiment, an anti-C5 antibody described herein comprises a heavy chain CDR2 comprising, or consisting of, the following amino acid sequence:

EILPGSGHTEYTENFKD (SEQ ID NO:18).

In another embodiment, the antibody binds to human C5 at pH 7.4 and 25C with an affinity dissociation constant ($K_D$) that is in the range 0.1 nM $\leq K_D \leq$ 1 nM. In another embodiment, the antibody binds to human C5 at pH 6.0 and 25C with a $K_D \geq$ 10 nM. In yet another embodiment, the $K_D$ of the antibody or antigen-binding fragment thereof for human C5 at pH 6.0 at 25C)/($K_D$ of the antibody or antigen-binding fragment thereof for human C5 at pH 7.4 at 25C of the antibody is greater than 25.

Another exemplary anti-C5 antibody is as described in US Patent Nos. 8,241,628 and 8,883,158. In one embodiment, the antibody, or antigen binding fragment thereof, comprises heavy chain CDR1, CDR2 and CDR3 domains having the sequences set forth in SEQ ID NOs: 21, 22 and 23, respectively, and light chain CDR1, CDR2 and CDR3 domains having the sequences set forth in SEQ ID NOs:24, 25 and 26, respectively. In another embodiment, the antibody, or antigen binding fragment thereof, comprises the $V_H$ region having the sequence set forth in SEQ ID NO:27, and the $V_L$ region having the sequence set forth in SEQ ID NO:28.

Another exemplary anti-C5 antibody is also described in US Patent Nos. 8,241,628 and 8,883,158. In one embodiment, the antibody, or antigen binding fragment thereof, comprises heavy chain CDR1, CDR2 and CDR3 domains having the sequences set forth in SEQ ID NOs:29, 30 and 31, respectively, and light chain CDR1, CDR2 and CDR3 domains having the sequences set forth in SEQ ID NOs:32, 33 and 34, respectively. In another embodiment, the antibody comprises the $V_H$ region having the sequence set forth in SEQ ID NO:35, and the $V_L$ region having the sequence set forth in SEQ ID NO:36.

Another exemplary anti-C5 antibody is described in US2016/0176954A1. In one embodiment, the antibody, or antigen binding fragment thereof, comprises heavy chain CDR1, CDR2 and CDR3 domains having the sequences set forth in SEQ ID NOs:37, 38 and 39, respectively, and light chain CDR1, CDR2 and CDR3 domains having the sequences set forth in SEQ ID NOs:40, 41 and 42, respectively. In another embodiment, the antibody comprises the $V_H$ region having the sequence set forth in SEQ ID NO:43, and the $V_L$ region having the sequence set forth in SEQ ID NO:44.

Another exemplary anti-C5 antibody is described in Fukuzawa T. et al. (Sci. Rep., 7:1080, 2017). In one embodiment, the antibody or antigen binding fragment thereof comprises a heavy chain comprising SEQ ID NO:45 and a light chain comprising SEQ ID NO:46.

Another exemplary anti-C5 antibody is described in US2017/0355757. In one embodiment, the antibody comprises a heavy chain variable region comprising SEQ ID NO:47 and a light chain variable region comprising SEQ ID NO:48. In another embodiment, the antibody comprises a heavy chain comprising SEQ ID NO:49 and a light chain comprising SEQ ID NO:50.

The antibodies described herein can compete for binding with, and/or bind to the same epitope on C5 as any of the above-mentioned antibodies. The term "binds to the same epitope" with reference to two or more antibodies means that the antibodies bind to the same segment of amino acid residues, as determined by a given method. Techniques for determining whether antibodies bind to the "same epitope on C5" with the antibodies described herein include, for example, epitope mapping methods, such as, X-ray analysis of crystals of antigen: antibody complexes that provides atomic resolution of the epitope and hydrogen/deuterium exchange mass spectrometry (HDX-MS). Other methods monitor the binding of the antibody to peptide antigen fragments or variations of the antigen where loss of binding due to a modification of an amino acid residue within the antigen sequence is often considered an indication of an epitope component. In addition, computational combinatorial methods for epitope mapping can also be used. These methods rely on the ability of the antibody of interest to affinity isolate specific short peptides from combinatorial phage display peptide libraries. Antibodies having the same $V_H$ and $V_L$ or the same CDR1, CDR2 and CDR3 sequences are expected to bind to the same epitope.

Antibodies described herein can have, for example, at least about 90% variable region amino acid sequence identity with the above-mentioned antibodies (*e.g.,* at least about 90%, 91%, 92%, 93%, 94%, 95%, 96%, 97%, 98% or 99% variable region identity).

An anti-C5 antibody described herein can, in some embodiments, comprise a variant human Fc region that binds to human neonatal Fc receptor (FcRn) with greater affinity than that of the native human Fc region from which the variant human Fc region was derived. The Fc constant region can comprise, for example, one or more (*e.g.,* two, three, four, five, six, seven, eight or more) amino acid substitutions relative to the native human Fc region from which the variant human Fc region was derived. The substitutions can increase the binding affinity of an IgG antibody containing the variant Fc region to FcRn at pH 6.0, while maintaining the pH dependence of the interaction. Methods for testing whether one or more substitutions in the Fc region of an antibody increase the affinity of the Fc region for FcRn at pH 6.0 (while maintaining pH dependence of the interaction) are known in the art and exemplified in the working examples.

Substitutions that enhance the binding affinity of an antibody Fc region for FcRn are known in the art and include, *e.g.,* (1) the M252Y/S254T/T256E triple substitution (Dall'Acqua, W. et al., J. Biol. Chem., 281:23514-24, 2006); (2) the M428L or T250Q/M428L substitutions (Hinton, P. et al., J. Biol. Chem., 279:6213-6, 2004; Hinton, P. et al., J. Immunol., 176:346-56); and (3) the N434A or T307/E380A/N434A substitutions (Petkova, S. et al., Int. Immunol., 18:1759-69, 2006). Additional substitution pairings, for example, P257I/Q311I, P257I/N434H and D376V/N434H are described in, *e.g.,* Datta-Mannan, A. et al. (J. Biol. Chem., 282:1709-17, 2007). The disclosures of each of these references are incorporated herein by reference in its entireties.

In some embodiments, the constant region can comprise a substitution at EU amino acid residue 255 for valine, a substitution at EU amino acid residue 309 for asparagine, a substitution at EU amino acid residue 312 for isoleucine and/or a substitution at EU amino acid residue 386.

The antibodies described herein can comprise a variant Fc region of no more than 30 (*e.g.,* no more than 29, 28, 27, 26, 25, 24, 23, 22, 21, 20, 19, 18, 17, 16, 15, 14, 13, 12, 11, 10, 9, 8, 7, 6, 5, 4, 3 or 2) amino acid substitutions, insertions or deletions relative to the native constant region from which it was derived. In some embodiments, the variant Fc region comprises one or more amino acid substitutions selected from the group consisting of: M252Y, S254T, T256E, N434S, M428L, V259I, T250I and V308F. In some embodiments, the variant human Fc region comprises a methionine at position 428 and an asparagine at position 434, each in EU numbering. In some embodiments, the variant Fc region comprises a 428L/434S double substitution as described in, *e.g.,* U.S. Patent No. 8,088,376.

In some embodiments the precise location of substitutions can be shifted from the native human Fc region position as desired for antibody engineering. For example, the 428L/434S double substitution, when used in a IgG2/4 chimeric Fc, can correspond to 429L and 435S as in the M429L and N435S variants found in ravulizumab (ULTOMIRIS®).

An antibody described herein can comprise, for example, a constant region comprising a substitution at one or more amino acid positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434 or 436 (EU numbering) relative to the native human constant region. In some embodiments, the substitution is selected from the group consisting of: methionine for glycine at position 237; alanine for proline at position 238; lysine for serine at position 239; isoleucine for lysine at position 248; alanine, phenylalanine, isoleucine, methionine, glutamine, serine, valine, tryptophan or tyrosine for threonine at position 250; phenylalanine, tryptophan or tyrosine for methionine at position 252; threonine for serine at position 254; glutamic acid for arginine at position 255; aspartic acid, glutamic acid or glutamine for threonine at position 256; alanine, glycine, isoleucine, leucine, methionine, asparagine, serine, threonine or valine for proline at position 257; histidine for glutamic acid at position 258; alanine for aspartic acid at position 265; phenylalanine for aspartic acid at position 270; alanine or glutamic acid for asparagine at position 286; histidine for threonine at position 289; alanine for asparagine at position 297; glycine for serine at position 298; alanine for valine at position 303; alanine for valine at position 305; alanine, aspartic acid, phenylalanine, glycine, histidine, isoleucine, lysine, leucine, methionine, asparagine, proline, glutamine, arginine, serine, valine, tryptophan or tyrosine for threonine at position 307; alanine, phenylalanine, isoleucine, leucine, methionine, proline, glutamine or threonine for valine at position 308; alanine, aspartic acid, glutamic acid, proline or arginine for leucine or valine at position 309; alanine, histidine or isoleucine for glutamine at position 311; alanine or histidine for aspartic acid at position 312;lysine or arginine for leucine at position 314; alanine or histidine for asparagine at position 315; alanine for lysine at position 317; glycine for asparagine at position 325; valine for isoleucine at position 332; leucine for lysine at position 334; histidine for lysine at position 360; alanine for aspartic acid at position 376; alanine for glutamic acid at position 380; alanine for glutamic acid at position 382; alanine for asparagine or serine at position 384; aspartic acid or histidine for glycine at position 385; proline for glutamine at position 386; glutamic acid for proline at position 387; alanine or serine for asparagine at position 389; alanine for serine at position 424; alanine, aspartic acid, phenylalanine, glycine, histidine, isoleucine, lysine, leucine, asparagine, proline, glutamine, serine, threonine, valine, tryptophan or tyrosine for methionine at position 428; lysine for histidine at position 433; alanine, phenylalanine, histidine, serine, tryptophan or tyrosine for asparagine at position 434; and histidine for tyrosine or phenylalanine at position 436, all in EU numbering.

Suitable anti-C5 antibodies for use in the methods described herein, in some embodiments, comprise a heavy chain polypeptide comprising the amino acid sequence depicted in SEQ ID NO:14 and/or a light chain polypeptide comprising the amino acid sequence depicted in SEQ ID NO:11. Alternatively, the anti-C5 antibodies for use in the methods described herein, in some embodiments, comprise a heavy chain polypeptide comprising the amino acid sequence depicted in SEQ ID NO:20 and/or a light chain polypeptide comprising the amino acid sequence depicted in SEQ ID NO:11.

In one embodiment, the antibody binds to C5 at pH 7.4 and 25°C (and, otherwise, under physiologic conditions) with a $K_D$ that is at least 0.1 nM (*e.g.,* at least 0.15, 0.175, 0.2, 0.25, 0.275, 0.3, 0.325, 0.35, 0.375, 0.4, 0.425, 0.45, 0.475, 0.5, 0.525, 0.55, 0.575, 0.6, 0.625, 0.65, 0.675, 0.7, 0.725, 0.75, 0.775, 0.8, 0.825, 0.85, 0.875, 0.9, 0.925, 0.95 or 0.975 nM). In some embodiments, the $K_D$ of the anti-C5 antibody, or antigen binding fragment thereof, is no greater than 1 nM (*e.g.* no greater than 0.9, 0.8, 0.7, 0.6, 0.5, 0.4, 0.3, or 0.2 nM).

In other embodiments, the $K_D$ of the antibody for C5 at pH 6.0 at 25°C)/($K_D$ of the antibody for C5 at pH 7.4 at 25C is greater than 21 (*e.g.,* greater than 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 350, 400, 450, 500, 600, 700, 800, 900, 1000, 1500, 2000, 2500, 3000, 3500, 4000, 4500, 5000, 5500, 6000, 6500, 7000, 7500 or 8000).

An anti-C5 antibody described herein can have a serum half-life in humans that is, for example, at least 20 days (*e.g.,* at least 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 or 55 days). In another embodiment, the anti-C5 antibody described herein has a serum half-life in humans that is at least 40 days. In another embodiment, the anti-C5 antibody described herein has a serum half-life in humans that is approximately 43 days. In another embodiment, the anti-C5 antibody described herein has a serum half-life in humans that is between 39-48 days. Methods for measuring the serum half-life of an antibody are known in the art. In some embodiments, an anti-C5 antibody, or antigen binding fragment thereof, described herein has a serum half-life that is at least 20% greater than the serum half-life of eculizumab (e.g., at least 30, 35, 40, 45, 50, 55, 60, 65, 70, 75, 80, 85, 90, 95, 100, 125, 150, 175, 200, 250, 300, 400 or 500% greater than the serum half-life of eculizumab).

Antibodies that "compete with another antibody for binding to a target" refer to antibodies that inhibit (partially or completely) the binding of the other antibody to the target. Whether two antibodies compete with each other for binding to a target, *i.e.,* whether and to what extent one antibody inhibits the binding of the other antibody to a target, may be determined using known competition experiments. In certain embodiments, an antibody competes with, and inhibits binding of another antibody to a target by at least 10%, 20%, 30%, 40%, 50%, 60%, 70%, 80%, 90% or 100%. The level of inhibition or competition may be different depending on which antibody is the "blocking antibody." Competing antibodies bind to the same epitope, an overlapping epitope or to adjacent epitopes (*e.g.,* as evidenced by steric hindrance).

Anti-C5 antibodies or antigen-binding fragments thereof described herein, used in the methods described herein can be generated using a variety of art-recognized techniques. Monoclonal antibodies can be obtained by various techniques familiar to those skilled in the art. Briefly, spleen cells from an animal immunized with a desired antigen are commonly immortalized by fusion with a myeloma cell (Köhler, G. & Milstein, C., Eur. J. Immunol., 6:511-9, 1976). Alternative methods of immortalization include transformation with Epstein Barr Virus, oncogenes or retroviruses, or other methods known in the art. Colonies arising from single immortalized cells are screened for production of antibodies of the desired specificity and affinity for the antigen, and yield of the monoclonal antibodies produced by such cells can be enhanced by various techniques, including injection into the peritoneal cavity of a vertebrate host. Alternatively, one can isolate DNA sequences that encode a monoclonal antibody or a binding fragment thereof by screening a DNA library from human B cells (Huse, W. et al., Science, 246:1275-81, 1989).

II. Compositions

Compositions comprising an anti-C5 antibody, or antigen binding fragment thereof, as described herein, can be formulated as a pharmaceutical solution. Pharmaceutical compositions generally include a pharmaceutically acceptable carrier. As used herein, a "pharmaceutically acceptable carrier" refers to, and includes, any and all solvents, dispersion media, coatings, antibacterial and antifungal agents, isotonic and absorption delaying agents, and the like that are physiologically compatible. Compositions can include, for example, a pharmaceutically acceptable salt, *e.g.,* an acid addition salt or a base addition salt, sugars, carbohydrates, polyols and/or tonicity modifiers.

Compositions described herein can be formulated according to standard methods. Pharmaceutical formulation is a well-established art (Gennaro, "Remington: The Science and Practice of Pharmacy," 20th Edition, Lippincott, Williams & Wilkins (ISBN: 0683306472), 2000; Ansel et al., "Pharmaceutical Dosage Forms and Drug Delivery Systems," 7th Edition, Lippincott Williams & Wilkins Publishers (ISBN: 0683305727), 1999; and Kibbe, "Handbook of Pharmaceutical Excipients American Pharmaceutical Association," 3rd Edition (ISBN: 091733096X), 2000). In some embodiments, a composition can be formulated, for example, as a buffered solution at a suitable concentration and suitable for storage at 2-8°C (*e.g.,* 4°C). In some embodiments, a composition can be formulated for storage at a temperature below 0°C (*e.g.,* -20°C or -80°C). In some embodiments, the composition can be formulated for storage for up to 2 years (*e.g.,* 1 month, 2 months, 3 months, 4 months, 5 months, 6 months, 7 months, 8 months, 9 months, 10 months, 11 months, 1 year, 1½ years or 2 years) at 2-8°C (*e.g.,* 4°C). Thus, in some embodiments, the compositions described herein are stable in storage for at least 1 year at 2-8C (*e.g.,* 4°C).

The pharmaceutical compositions can be in a variety of forms. These forms include, *e.g.,* liquid, semi-solid and solid dosage forms, such as liquid solutions (*e.g.,* injectable and infusible solutions), dispersions or suspensions, tablets, pills, powders, liposomes and suppositories. The preferred form depends, in part, on the intended mode of administration and therapeutic application. Compositions containing a composition intended for systemic or local delivery, for example, can be in the form of injectable or infusible solutions. Accordingly, the compositions can be formulated for administration by a parenteral mode (*e.g.*, intravenous, subcutaneous, intraperitoneal or intramuscular injection). "Parenteral administration," "administered parenterally," and other grammatically equivalent phrases, as used herein, refer to modes of administration other than enteral and topical administration, usually by injection, and include, without limitation, intravenous, intranasal, intraocular, pulmonary, intramuscular, intraarterial, intrathecal, intracapsular, intraorbital, intracardiac, intradermal, intrapulmonary, intraperitoneal, transtracheal, subcutaneous, subcuticular, intraarticular, subcapsular, subarachnoid, intraspinal, epidural, intracerebral, intracranial, intracarotid and intrasternal injection and infusion.

In some embodiments, the anti-C5 antibody, or antigen binding fragment thereof, is formulated as a pharmaceutical solution and administered intravenously.

In some embodiments, the composition comprises ravulizumab (ULTOMIRIS®) for injection. In one embodiment, the injection is a sterile, clear to translucent, slightly whitish color, preservative-free solution for intravenous use. In another embodiment, each single-dose vial contains 300 mg ravulizumab (ULTOMIRIS®) for injection at a concentration of 10 mg/mL with a pH of 7.0. In another embodiment, ravulizumab (ULTOMIRIS®) for injection requires dilution to a final concentration of 5 mg/mL. In another embodiment, each mL further comprises polysorbate 80 (0.2 mg; vegetable origin), sodium chloride (8.77 mg), sodium phosphate dibasic (1.78 mg), sodium phosphate monobasic (0.46 mg) and water.

III. Methods of Treatment

Provided herein are methods for treating CM-TMA in a human patient, comprising administering to the patient an anti-C5 antibody, or antigen binding fragment thereof (e.g., ravulizumab (ULTOMIRIS®)). As used herein, the term "subject" or "patient" is a human patient (*e.g.,* a patient having CM-TMA).

In some embodiments, the patient with CM-TMA excludes patients with aHUS, including postpartum aHUS, or any known gene mutation that causes aHUS. In some embodiments, the patient with CM-TMA is not a patient with chronic kidney disease (CKD). In some embodiments, the patient with CM-TMA is not a patient who has developed TMA due to hematopoietic stem cell transplantation (HSCT-TMA). In some embodiments, the patient with CM-TMA has no history of primary and secondary glomerular diseases other than lupus and/or primary antiphospholipid antibody syndrome (APS). In some embodiments, the patient has no history of Shiga toxin-producing *Escherichia coli* infection, *e.g.,* Shiga toxin-related hemolytic uremic syndrome (STEC-HUS). In some embodiments, the patient has no familial or acquired ADAMTS13 (a disintegrin and metalloproteinase with a thrombospondin type 1 motif, member 13) deficiency, *e.g.,* wherein the ADAMTS13 deficiency is attributed to activity of less than 5%. In some embodiments, the patient is negative on a direct Coombs test (test for identifying hemolytic anemia). In some embodiments, the patient's kidney biopsy is negative for interstitial fibrosis tubular atrophy, glomerulosclerosis, or crescent formation of at least 50%. In some embodiments, the patient is not a transplant patient with evidence of cellular/antibody-mediated graft rejection (AMR).

Complement-mediated thrombotic microangiopathy (CM-TMA) is a clinical disorder driven by the generation of excess complement. It is characterized by thrombocytopenia (a condition characterized by abnormally low levels of platelets, also known as thrombocytes, in the blood) and microangiopathic hemolytic anemia (a subgroup of hemolytic anemia (loss of red blood cells through destruction) caused by factors in the small blood vessels) with microvascular thrombosis (pathological occlusion of microvessels by fibrin- and/or platelet-rich thrombi) resulting in systemic organ damage (TMA). One form of CM-TMA, atypical hemolytic uremic syndrome (aHUS), is characterized by pathologic complement activation due to the loss of the natural regulators of the complement system, which results in systemic endothelial and organ damage.

aHUS can be genetic, acquired, or idiopathic. Hereditable forms of aHUS can be associated with mutations in a number of human complement components including, e.g., complement factor H (CFH), membrane cofactor protein (MCP), complement factor I (CFI), C4b-binding protein (C4BP), complement factor B (CFB), and complement component 3 (C3) (see, *e.g.,* Caprioli et al. (2006) Blood 108:1267-1279). Certain mutations in the gene encoding CD55, though not yet implicated in aHUS, are associated with the severity of aHUS (see, *e.g.,* Esparza-Gordillo et al. (2005) Hum Mol Genet 14:703-712). aHUS can be considered genetic when two or more (e.g., three, four, five, or six or more) members of the same family are affected by the disease at least six months apart and exposure to a common triggering agent has been excluded, or when one or more aHUS-associated gene mutations (e.g., one or more mutations in CFH, MCP/CD46, CFB, or CFI) are identified in a subject. For example, a subject can have CFH-associated aHUS, CFB-associated aHUS, CFI-associated aHUS, or MCP-associated aHUS. Up to 30% of genetic aHUS is associated with mutations in CFH, 12% with mutations in MCP, 5-10% with mutations in CFI, and less than 2% with mutations in CFB. Genetic aHUS can be multiplex (i.e., familial; two or more affected family members) or simplex (i.e., a single occurrence in a family). aHUS can be considered acquired when an underlying environmental factor (*e.g.,* a drug, systemic disease, or viral or bacterial agents that do not result in Shiga-like exotoxins) or trigger can be identified. aHUS can be considered idiopathic when no trigger (genetic or environmental) is evident.

Laboratory tests can be performed to determine whether a human subject has thrombocytopenia, microangiopathic hemolytic anemia, or acute renal insufficiency. Thrombocytopenia can be diagnosed by a medical professional as one or more of: (i) a platelet count that is less than 150,000/mm$^3$ (e.g., less than 60,000/mm$^3$); (ii) a reduction in platelet survival time that is reduced, reflecting enhanced platelet disruption in the circulation; and (iii) giant platelets observed in a peripheral smear, which is consistent with secondary activation of thrombocytopoiesis. Microangiopathic hemolytic anemia can be diagnosed by a medical professional as one or more of: (i) hemoglobin concentrations that are less than 10 mg/dL (e.g., less than 6.5 mg/dL); (ii) increased serum lactate dehydrogenase (LDH) concentrations (>460 U/L); (iii) hyperbilirubinemia, reticulocytosis, circulating free hemoglobin, and low or undetectable haptoglobin concentrations; and (iv) the detection of fragmented red blood cells (schistocytes) with the typical aspect of burr or helmet cells in the peripheral smear together with a negative Coombs test. See, e.g., Kaplan et al. (1992) "Hemolytic Uremic Syndrome and Thrombotic Thrombocytopenic Purpura," Informa Health Care (ISBN 0824786637) and Zipfel (2005) "Complement and Kidney Disease," Springer (ISBN 3764371668). Blood concentrations of C3 and C4 can also be used as a measure of complement activation or dysregulation. In addition, a subject's condition can be further characterized by identifying the subject as harboring one or more mutations in a gene associated with aHUS such as CFI, CFB, CFH, or MCP (*supra*). Suitable methods for detecting a mutation in a gene include, e.g., DNA sequencing and nucleic acid array techniques. See, e.g., Breslin et al. (2006) Clin Am Soc Nephrol 1:88-99 and Goicoechea de Jorge et al. (2007) Proc Natl Acad Sci USA 104:240-245.

In one aspect of the invention, the CM-TMA treated according to the methods described herein is associated with a trigger (also referred to as "secondary TMA"). As used herein, a "trigger" is an event, situation, or condition which causes CM-TMA to occur.

In one embodiment, the CM-TMA trigger is an autoimmune condition or event. Exemplary autoimmune conditions include, but are not limited to: acquired aplastic anemia, acute disseminated encephalomyelitis (ADEM), acute hemorrhagic leukoencephalitis (AHLE) / Hurst's disease, gammaglobulinemia, (primary), alopecia areata, ankylosing spondylitis (AS), anti-NMDA receptor encephalitis, antiphospholipid syndrome (APS), arteriosclerosis, autism spectrum disorders (ASD), autoimmune Addison's disease (AAD), autoimmune dysautonomia / Autoimmune autonomic ganglionopathy (AAG), autoimmune encephalitis, autoimmune gastritis, autoimmune hemolytic anemia (AIHA), autoimmune hepatitis (AIH), autoimmune hyperlipidemia, autoimmune hypophysitis / lymphocytic hypophysitis, autoimmune inner ear disease (AIED), autoimmune lymphoproliferative syndrome (ALPS), autoimmune myocarditis, autoimmune oophoritis, autoimmune orchitis, autoimmune pancreatitis (AIP) / Immunoglobulin G4-Related Disease (IgG4-RD), autoimmune polyglandular syndromes (Types I, II, & III), autoimmune progesterone dermatitis, autoimmune sudden sensorineural hearing loss (SNHL), balo disease, behçet's disease, birdshot chorioretinopathy / birdshot uveitis, bullous pemphigoid, castleman disease, celiac disease, chagas disease, chronic fatigue syndrome (CFS) / myalgic encephalomyelitis (ME), chronic inflammatory demyelinating polyneuropathy (CIDP), chronic Lyme disease / post-treatment Lyme disease syndrome (PTLDS), chronic urticaria (CU), churg-Strauss syndrome / eosinophilic granulomatosis with polyangiitis (EGPA), cicatricial pemphigoid, cogan's syndrome, cold agglutinin disease, CREST syndrome / limited cutaneous systemic sclerosis, crohn's disease (CD), cronkhite-Canada syndrome (CSS), cryptogenic organizing pneumonia (COP), dermatitis herpetiformis, dermatomyositis, type 1 diabetes, discoid lupus, eressler's syndrome / postmyocardial infarction / postpericardiotomy syndrome, endometriosis, eosinophilic fasciitis, erythema nodosum, essential mixed cryoglobulinemia, evans syndrome, fibrosing alveolitis, giant cell arteritis / temporal arteritis / Horton's disease,glomerulonephritis, goodpasture's syndrome / anti-GBM/anti-TBM disease, granulomatosis with polyangiitis (GPA) / Wegener's granulomatosis, grave's disease, guillain-barré syndrome (GBS), hashimoto's thyroiditis / chronic lymphocytic thyroiditis / autoimmune thyroiditis, henoch-Schönlein purpura / IgA vasculitis, herpes gestationis / pemphigoid gestationis, hypogammaglobulinemia, IgA nephropathy / Berger's disease, immune thrombocytopenia (ITP) / autoimmune thrombocytopenic purpura, interstitial cystitis, juvenile idiopathic arthritis, kawasaki disease, lambert-eaton myasthenic syndrome (LEMS), leukocytoclastic vasculitis, lichen planus, lichen sclerosus, ligneous conjunctivitis, linear IgA disease (LAD) / linear IgA bullous dermatosis (LABD), lupus nephritis, ménière's disease, microscopic polyangiitis (MPA), mixed connective tissue disease (MCTD), mooren's ulcer, mucha-Habermann disease, multiple sclerosis (MS), myasthenia gravis (MG), neuromyelitis optica (NMO) / Devic's disease, narcolepsy, non-length-dependent small fiber sensory neuropathy (SFSN), ocular cicatricial pemphigoid, opsoclonus-myoclonus syndrome (OMS), palindromic rheumatism, palmoplantar pustulosis (type of psoriasis), paraneoplastic cerebellar degeneration, paraneoplastic pemphigus, paroxysmal nocturnal hemoglobinuria (PNH), peripheral uveitis / pars planitis, parsonage turner syndrome, pemphigus foliaceus, pemphigus vulgaris, pernicious anemia, POEMS syndrome, polyarteritis nodosa, polymyalgia rheumatica, polymyositis, postural orthostatic tachycardia syndrome (POTS), primary biliary cholangitis (PBC) / primary biliary cirrhosis, primary sclerosing cholangitis (PSC), psoriasis, psoriatic arthritis, pulmonary fibrosis, idiopathic (IPF), pure red cell aplasia, pyoderma gangrenosum, raynaud's phenomenon, reactive arthritis / Reiter's syndrome, reflex sympathetic dystrophy syndrome, relapsing polychondritis, leg syndrome / Willis-Ekbom disease, rheumatic fever, rheumatoid arthritis, sarcoidosis, schmidt syndrome / autoimmune polyendocrine syndrome type II, systemic sclerosis, scleritis, scleroderma, serpiginous choroidopathy, sjögren's syndrome, stiff person syndrome (SPS), systemic lupus erythematosus (SLE), subacute bacterial endocarditis (SBE), sydenham's chorea, sympathetic ophthalmia, takayasu's arteritis (vasculitis), testicular autoimmunity (vasculitis / orchitis), tolosa-Hunt syndrome, transverse myelitis (TM), tubulointerstitial nephritis uveitis syndrome (TINU), ulcerative colitis (UC), undifferentiated connective tissue disease (UCTD), uveitis (anterior), uveitis (intermediate), uveitis (posterior), vasculitis, vitiligo, and vogt-Koyanagi-Harada syndrome (VKH). In one embodiment, the autoimmune trigger is lupus nephritis. In another embodiment, the autoimmune trigger is systemic sclerosis.

In another embodiment, the CM-TMA trigger is an infection, such as a bacterial infection, viral infection, fungal infection, or parasitic infection. In one embodiment, the trigger is bacterial infection selected from the group consisting of strep throat, abacterial urinary tract infection (UTIs) (*e.g.,* often caused by coliform bacteria), bacterial food poisoning (*e.g.,* often caused by E. coli, Salmonella, or Shigella), bacterial cellulitis (*e.g.*, such as due to Staphylococcus aureus (MRSA),bacterial vaginosis, gonorrhea, chlamydia, syphilis, Clostridium difficile (C. diff), tuberculosis, whooping cough, pneumococcal pneumonia, bacterial meningitis, Lyme disease cholera, botulism, tetanus, and anthrax.

In another embodiment, the trigger is a viral infection selected from the group consisting of influenza (the flu), the common cold, measles, rubella, chickenpox, norovirus, polio, infectious mononucleosis (mono), herpes simplex virus (HSV), human papillomavirus (HPV) human immunodeficiency virus (HIV), viral hepatitis, which can include hepatitis A, B, C, D, and E, viral meningitis, West Nile Virus, rabies, ebola, and COVID-19.

In another embodiment, the trigger is a fungal infection selected from the group consisting of a yeast infection, ringworm, athlete's foot, thrush, aspergillosis, histoplasmosis. cryptococcus infection, and fungal meningitis.

In another embodiment, the trigger is a parasitic infection selected from the group consisting of malaria, toxoplasmosis, trichomoniasis, giardiasis, tapeworm infection, roundworm infection, lice, scabies, leishmaniasis, and river blindness.

In another embodiment, the CM-TMA trigger is a transplant. In one embodiment, the trigger is a bone marrow transplant. In another embodiment, the trigger is a solid organ transplant (*e.g.,* selected from the group consisting of a kidney, pancreas, liver, heart, and small bowel transplant).

In another embodiment, the CM-TMA trigger is one or more drugs. In some embodiments, the TMA is triggered by a drug with an immune-mediated mode of action (e.g., quinine). In some embodiments, the TMA is triggered by a drug with a toxic mode of action (e.g., cyclosporine or tacrolimus). In specific embodiments, the TMA is triggered by a drug selected from clopidogrel, cyclosporine, estrogen/progesterone, gemcitabine, interferons, mitomycin, quinine, tacrolimus, ticlopidine, or a combination thereof.

In another embodiment, the CM-TMA trigger is malignant hypertension. Malignant hypertension is extremely high blood pressure (*e.g.,* above 180/120) that develops rapidly and causes some type of organ damage.

As used herein, "effective treatment" refers to treatment producing a beneficial effect, *e.g.,* amelioration of at least one symptom of a disease or disorder. A beneficial effect can take the form of an improvement over baseline, *i.e.,* an improvement over a measurement or observation made prior to initiation of therapy according to the method. For example, effective treatment may refer to alleviation of one more symptoms selected from the group consisting of a reduction or cessation in thrombocytopenia, microangiopathic hemolytic anemia, and/or microvascular thrombosis compared to baseline.

The term "effective amount" refers to an amount of an agent that provides the desired biological, therapeutic, and/or prophylactic result. That result can be reduction, amelioration, palliation, lessening, delaying, and/or alleviation of one or more of the signs, symptoms, or causes of a disease, or any other desired alteration of a biological system. In one example, an "effective amount" is the amount of anti-C5 antibody, or antigen binding fragment thereof, clinically proven to alleviate at least one symptom of CM-TMA. An effective amount can be administered in one or more administrations.

In one embodiment, a method of treating a human patient with CM-TMA is provided, the method comprising administering to the patient an effective amount of an anti-C5 antibody, or antigen binding fragment thereof, wherein the anti-C5 antibody, or antigen binding fragment thereof, comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively. In another embodiment, the antibody further comprises a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434 of a native human IgG Fc constant region, each in EU numbering.

In one embodiment, the anti-C5 antibody, or antigen binding fragment is administered at a fixed dose. For example, in one embodiment, the anti-C5 antibody, or antigen binding fragment is administered at a dose of 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 40 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg or 11000 mg, without regard to the patient's weight.

In another embodiment, the anti-C5 antibody, or antigen binding fragment thereof, (e.g., ravulizumab (ULTOMIRIS®)) is administered at a dose of 1200mg, 2400 mg, 2700 mg, 3000 mg, 3300 mg or 3600 mg.

In another embodiment, the dose of the anti-C5 antibody, or antigen binding fragment is based on the weight of the patient. For example, in one embodiment, 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg, or 11000 mg of the anti-C5 antibody, or antigen binding fragment is administered to a patient weighing ≥ 30 to < 40 kg. In another embodiment, 1200 mg or 2700 mg of the anti-C5 antibody, or antigen binding fragment thereof, (*e.g.,* ravulizumab (ULTOMIRIS®)) is administered to a patient weighing ≥ 30 to < 40 kg.

In another embodiment, 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg, or 11000 mg of the anti-C5 antibody, or antigen binding fragment is administered to a patient weighing ≥ 40 to < 60 kg. In another embodiment, 2400 mg or 3000 mg of the anti-C5 antibody, or antigen binding fragment thereof, (e.g., ravulizumab (ULTOMIRIS®)) is administered to a patient weighing ≥ 40 to < 60 kg.

In another embodiment, 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg, or 11000 mg of the anti-C5 antibody, or antigen binding fragment thereof, is administered to a patient weighing ≥ 60 to < 100 kg. In another embodiment, 2700 mg or 3300 mg of the anti-C5 antibody, or antigen binding fragment thereof, (*e.g.,* ravulizumab (ULTOMIRIS®)) is administered to a patient weighing ≥ 60 to < 100 kg.

In another embodiment, 10 mg, 20 mg, 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, 4200 mg, 4300 mg, 4400 mg, 4500 mg, 4600 mg, 4700 mg, 4800 mg, 4900 mg, 5000 mg, 5100 mg, 5200 mg, 5300 mg, 5400 mg, 5500 mg, 5600 mg, 5700 mg, 5800 mg, 5900 mg, 6000 mg, 6100 mg, 6200 mg, 6300 mg, 6400 mg, 6500 mg, 6600 mg, 6700 mg, 6800 mg, 6900 mg, 7000 mg, 7100 mg, 7200 mg, 7300 mg, 7400 mg, 7500 mg, 7600 mg, 7700 mg, 7800 mg, 7900 mg, 8000 mg, 8100 mg, 8200 mg, 8300 mg, 8400 mg, 8500 mg, 8600 mg, 8700 mg, 8800 mg, 8900 mg, 9000 mg, 9100 mg, 9200 mg, 9300 mg, 9400 mg, 9500 mg, 9600 mg, 9700 mg, 9800 mg, 9900 mg, 10000 mg, 10100 mg, 10200 mg, 10300 mg, 10400 mg, 10500 mg, 10600 mg, 10700 mg, 10800 mg, 10900 mg, or 11000 mg of the anti-C5 antibody, or antigen binding fragment, is administered to a patient weighing > 100 kg. In another embodiment, 3000 mg or 3600 mg of the anti-C5 antibody, or antigen binding fragment thereof, (*e.g.,* ravulizumab (ULTOMIRIS®)) is administered to a patient weighing > 100 kg.

In another embodiment, a method of treating a human patient with CM-TMA (*e.g.,* CM-TMA associated with a trigger, such as an autoimmune condition, an infection, a transplant, one or more drugs, or malignant hypertension) is provided, the method comprising administering to the patient an anti-C5 antibody, or antigen binding fragment thereof (*e.g.*, ravulizumab (ULTOMIRIS®):

(a) once on Day 1 at a dose of: 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing > 100 kg; and
(b) on Day 15 and every eight weeks thereafter at a dose of 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

In another embodiment, a method of treating a human patient with CM-TMA (e.g., CM-TMA associated with a trigger, such as an autoimmune condition, an infection, a transplant, one or more drugs, or malignant hypertension) is provided, wherein the anti-C5 antibody, or antigen binding fragment thereof, (*e.g.,* ravulizumab (ULTOMIRIS®)) is administered to a patient weighing ≥ 30 to < 40 kg:

(a) once on Day 1 at a dose of 1200 mg; and
(b) on Day 15 and every eight weeks thereafter at a dose of 2700 mg.

In another embodiment, a method of treating a human patient with CM-TMA (e.g., CM-TMA associated with a trigger, such as an autoimmune condition, an infection, a transplant, one or more drugs, or malignant hypertension) is provided, wherein the anti-C5 antibody, or antigen binding fragment thereof, (*e.g.,* ravulizumab (ULTOMIRIS®)) is administered to a patient weighing ≥ 40 to < 60 kg:

(a) once on Day 1 at a dose of 2400 mg; and
(b) on Day 15 and every eight weeks thereafter at a dose of 3000 mg.

In another embodiment, a method of treating a human patient with CM-TMA (*e.g.,* CM-TMA associated with a trigger, such as an autoimmune condition, an infection, a transplant, one or more drugs, or malignant hypertension) is provided, wherein the anti-C5 antibody, or antigen binding fragment thereof, (e.g., ravulizumab (ULTOMIRIS®)) is administered to a patient weighing ≥ 60 to < 100 kg:

(a) once on Day 1 at a dose of 2700 mg; and
(b) on Day 15 of the administration cycle and every eight weeks thereafter at a dose of 3300 mg.

In another embodiment, a method of treating a human patient with CM-TMA (*e.g.,* CM-TMA associated with a trigger, such as an autoimmune condition, an infection, a transplant, one or more drugs, or malignant hypertension) is provided, wherein the anti-C5 antibody, or antigen binding fragment thereof, (e.g., ravulizumab (ULTOMIRIS®)) is administered to a patient weighing ≥ 100 kg:

(a) once on Day 1 at a dose of 3000 mg; and
(b) on Day 15 and every eight weeks thereafter at a dose of 3600 mg.

In another embodiment, a method of treating a human patient with CM-TMA (*e.g.,* CM-TMA associated with a trigger, such as an autoimmune condition, an infection, a transplant, one or more drugs, or malignant hypertension) is provided, the method comprising administering to the patient an effective amount of an anti-C5 antibody, or antigen binding fragment thereof, wherein the anti-C5 antibody, or antigen binding fragment thereof, comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively, and a variant human Fc region that binds to human neonatal Fc receptor (FcRn), wherein the variant human Fc CH3 region comprises Met-429-Leu and Asn-435-Ser substitutions at residues corresponding to methionine 428 and asparagine 434 of a native human IgG Fc region, each in EU numbering, and wherein the anti-C5 antibody, or antigen binding fragment thereof, is administered to the patient:

(a) once on Day 1 at a dose of: 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing > 100 kg; and
(b) on Day 15 and every eight weeks thereafter at a dose of 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

In another embodiment, the anti-C5 antibody, or antigen binding fragment, is administered at a milligram per kilogram (mg/kg) dose. For example, in one embodiment, the anti-C5 antibody, or antigen binding fragment thereof, is administered at a dose of 0.1 mg/kg, 0.25 mg/kg, 0.5 mg/kg, 0.75 mg/kg, 1.0 mg/kg, 1.25 mg/kg, 1.50 mg/kg, 1.75 mg/kg, 2.0 mg/kg, 2.25 mg/kg, 2.50 mg/kg, 2.75 mg/kg, 3.0 mg/kg, 3.25 mg/kg, 3.50 mg/kg, 3.75 mg/kg, 4.0 mg/kg, 4.25 mg/kg, 4.50 mg/kg, 4.75 mg/kg, 5.0 mg/kg, 5.25 mg/kg, 5.50 mg/kg, 5.75 mg/kg, 6.0 mg/kg, 6.25 mg/kg, 6.50 mg/kg, 6.75 mg/kg, 7.0 mg/kg, 7.25 mg/kg, 7.50 mg/kg, 7.75 mg/kg, 8.0 mg/kg, 8.25 mg/kg, 8.50 mg/kg, 8.75 mg/kg, 9.0 mg/kg, 9.25 mg/kg, 9.50 mg/kg, 9.75 mg/kg, 10.0 mg/kg, 11.25 mg/kg, 11.50 mg/kg, 11.75 mg/kg, 12.0 mg/kg, 12.25 mg/kg, 12.50 mg/kg, 12.75 mg/kg, 13.0 mg/kg, 13.25 mg/kg, 13.50 mg/kg, 13.75 mg/kg, 14.0 mg/kg, 14.25 mg/kg, 14.50 mg/kg, 14.75 mg/kg, 15.0 mg/kg, 15.25 mg/kg, 15.50 mg/kg, 15.75 mg/kg, 16.0 mg/kg, 16.25 mg/kg, 16.50 mg/kg, 16.75 mg/kg, 17.0 mg/kg, 17.25 mg/kg, 17.50 mg/kg, 17.75 mg/kg, 18.0 mg/kg, 18.25 mg/kg, 18.50 mg/kg, 18.75 mg/kg, 19.0 mg/kg, 19.25 mg/kg, 19.50 mg/kg, 19.75 mg/kg, 20.0 mg/kg, 20.25 mg/kg, 20.50 mg/kg, 20.75 mg/kg, 21.0 mg/kg, 21.25 mg/kg, 21.50 mg/kg, 21.75 mg/kg, 22.0 mg/kg, 22.25 mg/kg, 22.50 mg/kg, 22.75 mg/kg, 23.0 mg/kg, 23.25 mg/kg, 23.50 mg/kg, 23.75 mg/kg, 24.0 mg/kg, 24.25 mg/kg, 24.50 mg/kg, 24.75 mg/kg, or 25.0 mg/kg.

In one embodiment, the anti-C5 antibody, or antigen binding fragment is administered once per week, twice per week, three times per week, four times per week, five times per week, six times per week, or daily. In another embodiment, anti-C5 antibody, or antigen binding fragment, is administered once every two weeks, once every three weeks, once every four weeks, once every five weeks, once every six weeks, once every seven weeks, once every eight weeks, once every nine weeks, once every ten weeks, once every eleven weeks, or once every twelve weeks. In another embodiment, the anti-C5 antibody, or antigen binding fragment, is administered at a loading dose on Day 1, followed by a different maintenance dose on Day 15 and every eight weeks thereafter.

In another embodiment, the anti-C5 antibody, or antigen binding fragment thereof, is administered for one or more administration cycles. In one embodiment, the administration cycle is 26 weeks. In another embodiment, the treatment comprises at least 1, 2, 3, 4, 5, 6, 7, 8, 9, 10, or 11 cycles. In another embodiment, the patient is treated for about 1, 2, 3, 4, 5, or 6 months. In another embodiment, the treatment continues for the lifetime of the human patient.

The anti-C5 antibody, or antigen binding fragment, can be administered via any suitable means. In one embodiment, the anti-C5 antibody, or antigen binding fragment (e.g., ravulizumab (ULTOMIRIS®)), is administered intravenously. In another embodiment, the anti-C5 antibody, or antigen binding fragment, is administered subcutaneously.

In some embodiments, the patients treated according to the methods described herein have been vaccinated against meningococcal infections within 3 years prior to, or at the time of, initiating treatment. In one embodiment, patients who received treatment less than 2 weeks after receiving a meningococcal vaccine are also treated with appropriate prophylactic antibiotics until 2 weeks after vaccination. In another embodiment, patients treated according to the methods described herein are vaccinated against meningococcal serotypes A, C, Y, W135, and/or B.

In some embodiments, the patients treated according to the methods have TMA associated with lupus nephritis, systemic sclerosis, or solid organ transplant. In one embodiment, these patients are vaccinated against Haemophilus influenzae type b (Hib) and Streptococcus pneumoniae prior to treatment.

In another aspect, the treatment regimens described are sufficient to maintain particular serum trough concentrations of the anti-C5 antibody, or antigen binding fragment thereof. The treatment can maintain, for example, a serum trough concentration of the anti-C5 antibody, or antigen binding fragment thereof, of 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, 205, 210, 215, 220, 225, 230, 240, 245, 250, 255, 260, 265, 270, 280, 290, 300, 305, 310, 315, 320, 325, 330, 335, 340, 345, 350, 355, 360, 365, 370, 375, 380, 385, 390, 395 or 400 μg/mL or greater. In one embodiment, the treatment maintains a serum trough concentration of the anti-C5 antibody, or antigen binding fragment thereof, of 100 μg/mL or greater. In another embodiment, the treatment maintains a serum trough concentration of the anti-C5 antibody, or antigen binding fragment thereof, of 150 μg/mL or greater. In another embodiment, the treatment maintains a serum trough concentration of the anti-C5 antibody, or antigen binding fragment thereof, of 200 μg/mL or greater. In another embodiment, the treatment maintains a serum trough concentration of the anti-C5 antibody, or antigen binding fragment thereof, of 250 μg/mL or greater. In another embodiment, the treatment maintains a serum trough concentration of the anti-C5 antibody, or antigen binding fragment thereof, of 300 μg/mL or greater. In another embodiment, the treatment maintains a serum trough concentration of the anti-C5 antibody, or antigen binding fragment thereof, of between 100 μg/mL and 200 μg/mL. In another embodiment, the treatment maintains a serum trough concentration of the anti-C5 antibody, or antigen binding fragment thereof, of about 175 µg/mL.

In another embodiment, to obtain an effective response, the anti-C5 antibody is administered to the patient in an amount and with a frequency to maintain at least 50 µg, 55µg, 60 µg, 65 µg, 70 µg, 75 µg, 80 µg, 85 µg, 90 µg, 95 µg, 100 µg, 105 µg, 110 µg, 115 µg, 120 µg, 125 µg, 130 µg, 135 µg, 140 µg, 145 µg, 150 µg, 155 µg, 160 µg, 165 µg, 170 µg, 175 µg, 180 µg, 185 µg, 190 µg, 195 µg, 200 µg, 205 µg, 210 µg, 215 µg, 220 µg, 225 µg, 230 µg, 235 µg, 240 µg, 245 µg, 250 µg, 255 µg or 260 µg of antibody per milliliter of the patient's blood. In another embodiment, the anti-C5 antibody is administered to the patient in an amount and with a frequency to maintain between 50 µg and 250 µg of antibody per milliliter of the patient's blood. In another embodiment, the anti-C5 antibody is administered to the patient in an amount and with a frequency to maintain between 100 µg and 200 µg of antibody per milliliter of the patient's blood. In another embodiment, the anti-C5 antibody is administered to the patient in an amount and with a frequency to maintain about 175 µg of antibody per milliliter of the patient's blood.

In another embodiment, to obtain an effective response, the anti-C5 antibody is administered to the patient in an amount and with a frequency to maintain a minimum free C5 concentration. The anti-C5 antibody can be administered, for example, to the patient in an amount and with a frequency to maintain a free C5 concentration of 0.2 µg/mL, 0.3 µg/mL, 0.4 µg/mL, 0.5 µg/mL or below. In another embodiment, the treatment described herein reduces free C5 concentration by greater than 99% throughout the treatment period. In another embodiment, the treatment reduces free C5 concentration greater than 99.5% throughout the treatment period.

In another aspect, the methods of treating CM-TMA described herein can be used alone or in combination with one more additional therapies and/or therapeutic agents. For example, in one embodiment, the method further comprises administering to the patient best supportive care. Best supportive care includes, but is not limited to, (a) transfusion support, (b) anti-infectives (e.g., antibiotics, antivirals, and antifungals), (c) renal replacement therapy (dialysis), (d) antihypertensive medications, (e) therapy for TMA associated with lupus nephritis or SSc-TMA, and/or (1) withdrawal or dose adjustment of the suspected agent for drug-induced TMA.

## IV. Outcomes

Provided herein are methods for treating CM-TMA in a patient comprising administering to the patient an anti-C5 antibody, or antigen binding fragment thereof (e.g., ravulizumab (ULTOMIRIS®)). The efficacy of the treatment methods provided herein can be assessed using any suitable means. In some embodiments, the treatment results in terminal complement inhibition.

In other embodiments, the treatment results in a normalization of platelet count without transfusion support and normalization of LDH levels.

In other embodiments, the treatment results in an improvement of eGFR of ≥ 30% compared to baseline. In other embodiments, the treatment results in a normalization of platelet count without transfusion support, normalization of LDH levels, and an improvement of eGFR of ≥ 30% compared to baseline. In other embodiments, the treatment results in a complete TMA response.

In other embodiments, the treatment results in LDH normalization (≤246 U/L). In other embodiments, the treatment results in LDH normalization (≤246 U/L) for at least 28 days (e.g., at least 28 days, 1 month, 2 months, 3 months, 4 months, 5 months, 6 months, 7 months, 8 months, 9 months, 10 months, 11 months, 1 year, or two years). In other embodiments, the treatment results in LDH normalization 5-12 days after initiating treatment. For example, in one embodiment, LDH normalization occurs 5, 6, 7, 8, 9, 10, 11, or 12 days after initiating treatment.

In other embodiments, the treatment results in a complete TMA response for at least 28 days (e.g., at least 28 days, 1 month, 2 months, 3 months, 4 months, 5 months, 6 months, 7 months, 8 months, 9 months, 10 months, 11 months, 1 year, or two years). In other embodiments, the treatment confers complete TMA response in the patient in fewer than about 60 days (e.g., 60, 59, 58, 57 56, 55, 54, 53, 52, 51, 50, 49, 48, 48, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, or 1 day(s)).

In other embodiments, the treatment results in hematological normalization.

In other embodiments, the treatment produces a reduction in the need for blood transfusions. In another embodiment, the treatment produces a greater than 70% increase in transfusion avoidance.

In other embodiments, the treatment produces a shift toward normal levels of one or more biomarkers selected from the group consisting of sTNF-RI, thrombomodulin, sVCAM-1, sC5b-9, C5a, factor Ba, and/or neutrophil gelatinase-associated lipocalin (NGAL).

In other embodiments, the treatment produces a change from baseline in quality of life, as assessed via the Functional Assessment of Chronic Illness Therapy (FACIT)-Fatigue Scale, the EuroQol 5-Dimension 5-Level (EQ-5D-5L) Scale, or the Kidney Disease Quality of Life instrument (KDQOL-36) Scale.

In other embodiments, the treatment prolongs survival of the patient (*e.g.*, by days, weeks, months, or years).

V. Kits and Unit Dosage Forms

Also provided herein are kits that include a pharmaceutical composition containing an anti-C5 antibody, or antigen binding fragment thereof (*e.g.,* any of those described herein previously) and a pharmaceutically acceptable carrier, in a therapeutically effective amount adapted for use in the methods described herein. The kits optionally also can include instructions, *e.g.,* comprising administration schedules, to allow a practitioner (*e.g.,* a physician, nurse or patient) to administer the composition contained therein to administer the composition to a patient having CM-TMA (*e.g.,* CM-TMA associated with a trigger, such as an autoimmune condition, an infection, a transplant, one or more drugs, or malignant hypertension). The kit also can include a syringe.

Optionally, the kits include multiple packages of the single-dose pharmaceutical compositions each containing an effective amount of the anti-C5 antibody, or antigen binding fragment thereof, for a single administration in accordance with the methods provided above. Instruments or devices necessary for administering the pharmaceutical composition(s) also may be included in the kits. For instance, a kit may provide one or more pre-filled syringes containing an amount of the anti-C5 antibody, or antigen binding fragment thereof.

In one embodiment, a kit for treating CM-TMA (*e.g.,* CM-TMA associated with a trigger, such as an autoimmune condition, an infection, a transplant, one or more drugs, or malignant hypertension) in a human patient is provided, the kit comprising: (a) a dose of an anti-C5 antibody, or antigen binding fragment thereof, wherein the anti-C5 antibody, or antigen binding fragment thereof, comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively; and (b) instructions for using the anti-C5 antibody, or antigen binding fragment thereof, in the methods described herein.

In another embodiment, a kit for treating CM-TMA (*e.g.,* CM-TMA associated with a trigger, such as an autoimmune condition, an infection, a transplant, one or more drugs, or malignant hypertension)in a human patient is provided, the kit comprising: (a) a dose of an anti-C5 antibody, or antigen binding fragment thereof, wherein the anti-C5 antibody, or antigen binding fragment thereof, comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively, and a variant human Fc region that binds to human neonatal Fc receptor (FcRn), wherein the variant human Fc CH3 region comprises Met-429-Leu and Asn-435-Ser substitutions at residues corresponding to methionine 428 and asparagine 434 of a native human IgG Fc region, each in EU numbering; and (b) instructions for using the anti-C5 antibody, or antigen binding fragment thereof, in the methods described herein.

In another embodiment, a kit for treating CM-TMA (*e.g.,* CM-TMA associated with a trigger, such as an autoimmune condition, an infection, a transplant, one or more drugs, or malignant hypertension) in a human patient is provided, the kit comprising: (a) a dose of ravulizumab (ULTOMIRIS®) and (b) instructions for using ravulizumab (ULTOMIRIS®) in the methods described herein.

Unless otherwise defined, all technical and scientific terms used herein have the same meaning as commonly understood by one of ordinary skill in the art to which the present disclosure belongs. Methods and materials are described herein for use in the present disclosure; other, suitable methods and materials known in the art can also be used. The materials, methods, and examples are illustrative only and not intended to be limiting. All publications, patent applications, patents, sequences, database entries (*e.g.,* PUBMED, NCBI or UNIPROT accession numbers), and other references mentioned herein are incorporated by reference in their entirety.

## EXAMPLE

**EXAMPLE 1: A Phase 3, Randomized, Double-blind, Placebo-Controlled, Multicenter Study to Evaluate the Efficacy and Safety of Ravulizumab (ULTOMIRIS®) in Adult Participants Who Have Thrombotic Microangiopathy Associated With a Trigger**

### A. Trial Design

A Phase 3, randomized, double-blind, placebo-controlled study of ravulizumab (ULTOMIRIS®) in addition to best supportive care (BSC) is conducted in adult participants (≥ 18 years of age) with TMA following a defined trigger. A schematic of the trial design is set forth in **FIG. 1.** All participants have acute severe renal dysfunction and a diagnosis of TMA based on protocol-defined criteria (*i.e.,* thrombocytopenia, microangiopathic hemolytic anemia, elevated lactate dehydrogenase (LDH), and severe renal dysfunction), which is associated with at least one trigger (*e.g.,* autoimmune, infection, solid organ transplant, drugs, or malignant hypertension), occurring ≤ 14 days prior to randomization.

The study consists of an up to 2-week Screening Period, a 26-week randomized Treatment Period, and a 26-week Post-treatment Follow-up Period. Thus, the total treatment duration is 26 weeks and the total study duration is up to 54 weeks.

Participants are screened for eligibility for up to 2 weeks during the Screening Period. Approximately 100 adult participants are randomized in a 1:1 ratio to receive either ravulizumab (ULTOMIRIS®) or placebo. Randomization is stratified by baseline dialysis status and by type of trigger.

During the 26-week Treatment Period, all participants receive a weight-based loading dose of ravulizumab (ULTOMIRIS®) or placebo on Day 1, followed by weight-based maintenance doses of ravulizumab (ULTOMIRIS®) or placebo on Day 15 and then once every 8 weeks (q8w) thereafter, as set forth in Table 1. All participants receive best supportive care (BSC) throughout the study.

**Table 1: Ravulizumab (ULTOMIRIS®) Weight Based Dosage Regimen**

| Body Weight Range[a] | Loading Dose (Day 1) | Maintenance Doses (Days 15, 71, and 127) |
|---|---|---|
| $\geq$30 kg to < 40 kg | 1200 mg | 2700 mg |
| $\geq$40 kg to < 60 kg | 2400 mg | 3000 mg |
| $\geq$60 kg to < 100 kg | 2700 mg | 3300 mg |
| $\geq$100 kg | 3000 mg | 3600 mg |
| [a] Dose is based on the last recorded study visit body weight. | | |

During the 26-week Post-Treatment Follow-up Period, participants continue to receive BSC, at the discretion of the Investigator, and are monitored for safety, TMA response, and clinical events of interest.

**B. Objectives and Endpoints**

The primary objective of the study is to assess the efficacy of ravulizumab (ULTOMIRIS®) in the treatment of participants with TMA, e.g., as assessed by the proportion of participants who achieve Complete TMA Response at Week 26.

Secondary objectives include: (1) characterizing TMA response (*e.g.,* based on time to Complete TMA Response, time to response for each TMA parameter, proportion of participants who achieve Hematologic Response at Week 26, proportion of participants who achieve Renal Response at Week 26, and proportion of participants with response in at least 1 TMA parameter by Week 26), (2) assessing impact on hemoglobin levels (e.g., based on proportion of participants with an increase of $\geq$ 2 grams in hemoglobin by Week 26), (3) evaluating change in kidney function (*e.g.,* based on change from baseline in estimated glomerular filtration rate (eGFR) at Week 26 and change from baseline in dialysis requirement at Week 26 and Week 52), (4) and assessing duration of Complete TMA Response and TMA Relapse (*e.g.,* based on proportion of participants with Complete TMA Response at Week 26 who maintain response at Week 52 and proportion of participants who have TMA Relapse during the study (among participants who achieved Complete TMA Response)).

Pharmacokinetic (PK), pharmacodynamic (PD) and immunogenicity objectives include: (1) assessing PK/PD of ravulizumab (ULTOMIRIS®) in participants with TMA (*e.g.,* based on (a) serum ravulizumab (ULTOMIRIS®) concentrations over time, (b) absolute values, change from baseline, and percentage change from baseline in serum free C5 concentrations over time, and (c) absolute values, change from baseline, and percentage change from baseline in serum total C5 concentrations over time) and (2) characterizing the potential for immunogenicity of ravulizumab (ULTOMIRIS®) in participants with TMA (e.g., based on incidence and titers of antidrug antibodies (ADAs) over time).

Safety objectives include characterizing the safety profile of ravulizumab (ULTOMIRIS®) in participants with TMA (*e.g.,* based on incidence of adverse events (AEs) and serious adverse events (SAEs) over time and the proportion of participants experiencing major adverse cardiovascular event (MACEs) from baseline through Week 26 and Week 52).

Additional objectives include assessing (1) improvement in participant reported QoL outcomes (*e.g.,* based on change in participant-reported outcomes as measured by EQ-5D-5L, FACIT-Fatigue, and KDQOL-36), (2) biomarkers, such as sC5b-9 and factor Ba, in blood and urine, as well as autoantibodies in participants with TMA, (3) complement pathway genetic mutations in participants with complement-mediated TMA (*e.g.,* based on incidence of complement dysregulation-related mutations) and (4) health resource utilization during the study (*e.g.*, based on number and duration of hospitalizations (including stays in intensive care unit, if applicable) and number of outpatient visits (including physician and emergency room visits).

**C. Endpoint Definitions**

Complete TMA Response is defined as the normalization of hematologic parameters (platelet count and LDH) and ≥ 30% improvement in estimated glomerular filtration rate (eGFR) from baseline (see Table 2). For Complete TMA Response, participants must have met all three TMA response parameters at two separate assessments obtained at least 24 hours apart, and any measurement in between. All intervals during which each criterion is met must overlap by at least one day.

**Table 2: Overview of Complete Thrombotic Microangiopathy Response**

| | TMA Response Parameter |
|---|---|
| Hematologic Response | •Normalization of platelet count without transfusion support during the prior 7 days<br>•Normalization of lactate dehydrogenase (LDH) |
| Renal Response | •Improvement in estimated glomerular filtration rate (eGFR) of ≥ 30% compared to baseline |

Hematologic Response is defined as the normalization of platelets without transfusion support during the prior 7 days and the normalization of LDH.

Renal Response is defined as an improvement in eGFR of ≥ 30% compared to baseline.

For participants that meet the criteria for TMA Complete Response, TMA Relapse is defined as occurrence of all of the following criteria: (1) Platelet count < 150,000/μL, (2) LDH > 1.5 × upper limit of normal (ULN), (3) Hemoglobin ≤ lower limit of normal (LLN), and (4) evidence of renal dysfunction due to TMA (e.g., worsening eGFR).

### D. Study Population

To be eligible to participate in the study, participants must meet all of the below criteria:

1. Must be ≥ 18 years of age at the time of signing the informed consent.
2. Body weight ≥ 30 kg at Screening.
3. Male and female: Female participants of childbearing potential and male participants must follow protocol specified contraception guidance.
4. Diagnosis of TMA within ≤ 14 days prior to Screening is associated with at least 1 of the following triggers: (a) autoimmune (lupus nephritis, systemic sclerosis associated TMA [SSc-TMA]), (b) infection, (c) solid organ transplant (kidney, pancreas, liver, heart, small bowel), (d) drugs, and (e) malignant hypertension.
5. Confirmation of all of the following laboratory findings during Screening Period based on central laboratory results: (a) platelet count < 150,000/μL, (b) lactate dehydrogenase ≥ 1.5 × ULN and hemoglobin ≤ LLN, and (c) acute kidney injury as defined by meeting 1 or more of the following criteria: acute decline in eGFR ≥ 50% from pre-TMA value (pre-TMA value must be ≤ 12 months prior to Screening); if pre-TMA eGFR in the previous 12 months is not available, new acute kidney injury with an eGFR ≤ 30 mL/min/1.73 m$^2$; and new initiation of dialysis due to TMA requiring > 1 session and for no more than 2 weeks prior to Screening.
6. Vaccinated against meningococcal infection (N meningitidis), within 3 years prior to, or at the time of, randomization. Participants who initiate study drug treatment less than 2 weeks after receiving a meningococcal vaccine must receive appropriate prophylactic antibiotics for at least 2 weeks after the vaccination. If participant cannot receive the meningococcal vaccine, then participant must receive antibiotic prophylaxis coverage against N meningitidis during the entire Treatment Period and for 8 months following the final dose of study drug.
7. Participants with TMA associated with lupus nephritis, systemic sclerosis, or solid organ transplant must be vaccinated against Haemophilus influenzae type b (Hib) and Streptococcus pneumoniae prior to randomization, according to current national/local vaccination guidelines
8. Capable of giving signed informed consent.

Participants are excluded from the study if any of the following criteria apply:

1. Diagnosis of aHUS, including postpartum aHUS, or any known gene mutation that causes aHUS.
2. Known CKD with eGFR ≤ 45 mL/min/1.73 m2 by CKD-EPI equation (Levey AS, et al., Ann Intern Med. 2009;150(9):604-612) due to any cause.
3. TMA due to hematopoietic stem cell transplantation ≤ 12 months of Screening.
4. Primary and secondary glomerular diseases other than lupus.
5. Diagnosis of primary antiphospholipid antibody syndrome.
6. Shiga toxin-producing Escherichia coli infections, including but not limited to, Shiga toxin-related hemolytic uremic syndrome.

7. Known familial or acquired a disintegrin and metalloproteinase with a thrombospondin type 1 motif, member 13' (ADAMTS13) deficiency (activity < 5%).

8. Positive direct Coombs test.

9. Diagnosis of disseminated intravascular coagulation (DIC) according to the International Society on Thrombosis and Haemostasis (ISTH) scoring criteria (*e.g.*, as set forth in Taylor et al., Thromb Haemost. 2001;86(5):1327-13300.

10. Presence of sepsis according to the Third International Consensus definition (Singer M, et al., JAMA. 2016;315(8):801-810) within 7 days prior to or during Screening.

11. Presence of monoclonal gammopathy, including but not limited to, multiple myeloma.

12. Known bone marrow insufficiency or failure evidenced by cytopenias.

13. Kidney biopsy (if available) showing interstitial fibrosis tubular atrophy, glomerulosclerosis, or crescent formation of $\geq$ 50%.

14. Among transplant recipients, presence of confirmed cellular and antibody-mediated graft rejection.

15. Life expectancy < 6 months, hospice, or palliative care.

16. Unresolved N meningitidis infection.

17. Human immunodeficiency virus (HIV) infection (evidenced by HIV-1 or HIV 2 antibody titer, or documented negative HIV-1/HIV-2 tests within 6 months prior to Screening.

18. History of malignancy within 5 years of Screening with the exception of nonmelanoma skin cancer or carcinoma in situ of the cervix that has been treated with no evidence of recurrence.

19. Known medical or psychological condition(s) or risk factor that, in the opinion of the Investigator, might interfere with the participant's full participation in the study, pose any additional risk for the participant, or confound the assessment of the participant or outcome of the study.

20. Hypersensitivity to any ingredient contained in the study drug, including hypersensitivity to murine proteins.

21. Use of any complement inhibitors within the past 3 years

22. Participation in another interventional treatment study for TMA or in any interventional treatment study with an unapproved therapy within 30 days before initiation of study drug on Day 1 of this study or within 5 half-lives of that investigational product, whichever is greater.

23. Pregnant, breastfeeding, or intending to conceive during the course of the study

**E. Study Drug**

Ravulizumab (ULTOMIRIS®) is formulated at pH 7.0 and is supplied in 30 mL single-use vials. Each vial of ravulizumab (ULTOMIRIS®) contains 300 mg of ravulizumab (ULTOMIRIS®) (10 mg/mL) in 10 mM sodium phosphate, 150 mM sodium chloride, 0.02% polysorbate 80, and water for injection. The comparator product (placebo) is formulated as a matching sterile, clear, colorless solution with the same buffer components, but without active ingredient. Additional details are presented in Table 3.

**Table 3: Study Drug**

| Intervention Name | Ravulizumab | Placebo |
|---|---|---|
| **Dose Formulation** | Vial | Vial |
| **Physical description** | Liquid solution practically free from particles | Liquid solution practically free from particles |
| **Unit Dose Strength** | 300 mg (10 mg/mL concentrated solution) | Placebo |
| **Route of Administration** | Intravenous (IV) infusion | Intravenous (IV) infusion |
| **Use** | Experimental | Placebo comparator |
| **Sourcing** | Provided centrally by Alexion or contracted manufacturing organization | Provided centrally by Alexion or contracted manufacturing organization. |
| **Packaging and Labeling** | Ravulizumab is provided in glass vials and stoppered with a butyl rubber stopper with aluminum overseal and flip-off cap. Ravulizumab is supplied in kits and labeled as required per country requirement. | Placebo is provided in glass vials and stoppered with a butyl rubber stopper with aluminum overseal and flip-off cap. Placebo is supplied in kits and labeled as required per country requirement. |

The dosing regimen consists of a loading dose followed by maintenance dosing administered q8w. The maintenance dosing is initiated 2 weeks after the loading dose administration.

Weight-based dosing based on the participant's body weight recorded at the day of the infusion visit (see Table 1). If the study intervention is prepared the day prior to the visit, the weight from the prior visit is used to determine the dose.

At the scheduled dosing visits, study drug is administered after all other tests and procedures have been completed, excluding the post-dose sample collections (pharmacokinetic [PK]/pharmacodynamic [PD]/biomarkers).

During the Treatment Period, participants are randomized 1: 1 to receive blinded doses of ravulizumab (ULTOMIRIS®) or placebo. Participants in the ravulizumab (ULTOMIRIS®) group receive a blinded loading dose of ravulizumab (ULTOMIRIS®) via intravenous infusion on Day 1, followed by a blinded maintenance dose at Week 2 then every eight weeks thereafter through the end of the Treatment Period. Participants in the placebo group receive a blinded matching placebo dose via intravenous infusion on Day 1, followed by a blinded matching placebo dose at Day 15, then every eight weeks thereafter through the end of the Treatment Period.

## F. Best Supportive Care and Concomitant Therapy

Participants in both treatment groups receive best supportive care (BSC) as background therapy. Best supportive care measures, include but are not limited to: (a) transfusion support, which should be provided as required per institutional guidelines and based on the participant's clinical condition, (b) anti-infectives (e.g., antibiotics, antivirals, and antifungals), (c) renal replacement therapy (dialysis), (d) antihypertensive medications, (e) therapy for TMA associated with lupus nephritis or SSc-TMA, and (f) withdrawal or dose adjustment of the suspected agent for drug-induced TMA.

Any medication or therapy (including over-the-counter or prescription medicines, vaccines, vitamins, and/or herbal supplements) deemed necessary for the participant's care during the study, or for the treatment of any adverse event, along with any other medications, other than those listed as disallowed medications, can be given at the discretion of the Investigator.

Participants are prohibited from receiving any of the following medications and therapies during the entire duration of study participation: (a) experimental interventions or therapies, (b) eculizumab or other agents that act on the complement pathway, and (c) therapeutic plasma exchange/plasma infusion.

## G. Vaccination and Antibiotic Prophylaxis

Due to its mechanism of action, the use of ravulizumab (ULTOMIRIS®) increases a participant's susceptibility to meningococcal infection due to N meningitidis. To reduce the risk of infection, all participants are vaccinated within 3 years prior to or at the time of the first infusion of study drug. Participants who have not been vaccinated prior to starting study drug for any reason, receive appropriate prophylactic antibiotics prior to and for at least 2 weeks after vaccination. Vaccines against serotypes A, C, Y, W135, and B where available, are recommended in preventing the commonly pathogenic meningococcal serotypes. Participants receive the complete primary vaccination series and be revaccinated if indicated according to current national vaccination guidelines. Vaccination may not be sufficient to prevent meningococcal infection.

Participants are administered prophylactic antibiotics for meningococcal infection until at least two weeks after vaccination if randomization occurs less than two weeks after initial vaccination. Consideration should be given per official guidance and local practice on the appropriate use of prophylactic antibacterial agents. All participants are monitored for early signs of meningococcal infection, evaluated immediately if infection is suspected, and treated with appropriate antibiotics, if necessary. Participants who cannot receive meningococcal vaccine receive antibiotic prophylaxis coverage against N meningitis during the entire Treatment Period and for eight months following the final dose of study drug.

Meningococcal serogroups ACWY and B vaccinations are required during screening for participants who do not meet criteria for previous vaccination. The vaccination series is completed during the study according to national and local vaccination schedule guidelines. Participants who have TMA associated with triggers of lupus nephritis, systemic sclerosis, or solid organ transplant are also vaccinated against Hib and S pneumoniae prior to randomization, unless previously vaccinated, according to current national/local vaccination guidelines.

## H. Biomarkers

Blood (whole blood, serum, and plasma) samples for biomarker research are collected from all participants at select time points specified. Biomarkers include, but are not limited to, assessments of the following: (1) vascular inflammation (e.g., shed tumor necrosis factor receptor I [sTNF-RI]), (2) endothelial damage and/or activation (e.g., thrombomodulin and shed vascular cell adhesion molecule 1 [sVCAM-1]), and (3) complement pathway dysregulation (e.g.,

soluble C5b-9 [sC5b-9] and factor Ba).

Urine samples for biomarker research are collected from all participants during Screening and at select time points. Biomarkers include, but are not limited to, assessments of the following: (1) complement pathway dysregulation (e.g., sC5b-9 and factor Ba) and (2) renal injury biomarkers (e.g., neutrophil gelatinase-associated lipocalin [NGAL]).

Blood and urine samples are collected, at select study sites, for exploratory Real Time Complement Activity (RTCA) during Screening and at select time points through the end of the Treatment Period (Day 183). The RTCA tests are performed using freshly collected whole blood dipotassium ethylenediaminetetraacetic acid (K2EDTA), plasma, and urine samples Blood and urine samples for RTCA are collected prior to administration of study drug on dosing days, if applicable.

Residual blood and urine samples from exploratory biomarkers, PK, PD, and immunogenicity, are stored for additional method developments of assays (e.g., prognostic and/or diagnostic tests related to the study drug target, disease process, pathways associated with disease state, other TMA or complement-related diseases, and/or mechanism of action of ravulizumab (ULTOMIRIS®)).

## I. Other Exploratory Assessments

Quality of life scales are administered. All assessments are administered in a quiet room. These assessments can be recorded on paper, if electronic devices are unavailable or cannot be used.

The following quality of life scales are administered:

(1) The Functional Assessment of Chronic Illness Therapy (FACIT) Fatigue scale, Version 4.0, which is a 13-item questionnaire that assesses self-reported fatigue and its impact upon daily activities and function over the preceding 7 days;

(2) The EuroQoL 5-Dimensions 5-Level (EQ-5D-5L), which is a self-assessed, standardized instrument to measure health related quality of life and has been used in a wide range of health conditions. The EQ 5D 5L comprises 5 dimensions, each describing a different aspect of health: mobility, self-care, usual activities, pain/discomfort, and anxiety/depression; and

(3) The Kidney Disease Quality of Life instrument 36 items (KDQOL-36™) (Section 10.9.3) is a 36-item kidney-specific health-related quality of life measure including Short-Form Health Survey 12 Item (SF-12) as generic core plus the burden of kidney disease, symptoms/problems of kidney disease, and effects of kidney disease scales.

## J. Statistical Considerations

The primary efficacy analysis tests the hypothesis that ravulizumab (ULTOMIRIS®) is superior to placebo based on the proportion of participants achieving Complete TMA Response during the 26-week Treatment Period. Hypothesis testing is 2-sided and performed at the 0.05 level of significance. The null and alternative hypotheses for the primary analysis are as follows:

$$H_0: P_{rav} = P_{placebo} \text{ vs. } HA: P_{rav} \neq P_{placebo}$$

where $P_{rav}$ is the proportion of participants achieving Complete TMA Response in the ravulizumab (ULTOMIRIS®) plus BSC group and $P_{placebo}$ is the proportion of participants achieving Complete TMA Response in the placebo plus BSC group.

The sample size determination was based on a 2-sided Fisher's exact test performed at a 2-sided significance level of 0.05, comparing the proportion of participants achieving Complete TMA Response at Week 26 in participants randomized to ravulizumab (ULTOMIRIS®) versus placebo. A sample size of 100 (50 participants per treatment group) has approximately 90% power to detect a statistically significant ($p \leq 0.05$) treatment difference of 35% in the proportion of responders at a 2-sided significance level of 0.05, assuming the responder rate is 35% with placebo plus BSC and 70% with ravulizumab (ULTOMIRIS®) plus BSC and an anticipated 10% drop-out rate (see, *e.g.*, Caires RA, et al., Transplant Proc. 2012;44(8):2388-2390; Humphreys BD, et al., Cancer. 2004;100(12):2664-2670; Lee C-S, et al., Nephrology. 2012;17(1):48-52; Schwarz A, et al., Am J Transplant. 2010;10(9):2017-2025; Song D, et al., Arthritis Res Ther. 2013;15(1):R12-R12; Waters, et al., J Pediatr. 2007;151(2):140-144; and Wu L-H, et al., Kidney Int Suppl. 2013;83(4):715-723).

The population sets used for analysis are defined in Table 4 as follows:

**Table 4: Population Sets**

| Population | Description |
|---|---|
| Enrolled Set | All consented participants meeting eligibility criteria. |
| Intent to Treat (ITT) | All randomized participants. |
| Safety Set | All participants who receive at least one dose of study drug. Participants are analyzed according to the study drug they actually receive for reporting exposure and safety data. |
| Pharmacokinetic/Pharmacodynamic (PK/PD) Analysis Set | All participants who receive at least one dose of study drug and who have evaluable PK/PD data |
| Per Protocol Set | To be defined in the Statistical Analysis Plan. |

Summary statistics are computed and displayed by treatment group and by visit, where applicable. Descriptive statistics for continuous variables minimally include the number of participants, mean, standard deviation (SD), minimum, median, and maximum. For categorical variables, frequencies and percentages are presented. Graphical displays are provided as appropriate. Analyses are performed using the SAS® software Version 9.4 or higher.

Efficacy analyses are performed using the ITT Set, the primary efficacy population. The primary analysis and selected secondary efficacy analyses are performed on the PP Set as sensitivity analyses as necessary.

The primary efficacy analysis tests the hypothesis that ravulizumab (ULTOMIRIS®) is superior to placebo based on the proportion of participants achieving Complete TMA Response during the 26-week Treatment Period. Participants must meet each TMA Response criterion at two separate assessments at least 24 hours apart, and any measurement in between. The primary efficacy analysis is performed at the end of the 26-week Treatment Period after all participants have completed 26 weeks or withdrawn early from the 26-week Treatment Period.

The primary analysis is based on a Cochran-Mantel-Haenszel test stratified by randomization stratification factors at a 5% significance level, comparing the proportion of participants achieving Complete TMA Response during the 26-week randomized Treatment Period between the two treatment groups in the ITT Set. The analysis is performed according to the randomized treatment assignment. For participants who withdraw from the study, data up to the time of withdrawal is used to assess Complete TMA Response.

Baseline value is defined as the average of the values from the assessments performed prior to the first dose of study drug (*i.e.,* results from Screening and the Day 1 visit). When a participant is on dialysis at baseline, then the baseline value is defined as above. If a participant is on dialysis during the entire 26 week Treatment Period, or through early discontinuation of study drug, then the change in eGFR is not calculated.

In addition, Complete TMA Response is summarized by treatment group over time by presenting the number and proportion of responders along with a 2-sided 95% confidence interval (CI) for each post-baseline time point.

As a sensitivity analysis, a 2-sided Fisher's exact test on the primary endpoint is performed. A re-randomization test to calculate the p-value is also explored. The p-values from sensitivity analyses for efficacy endpoints are for descriptive purpose only and not adjusted for multiplicity.

For time to Complete TMA Response, participants are assigned as responders at the time of their response and are censored at the earliest of their discontinuation time or at the end of available follow-up if they have not responded by then. Kaplan-Meier cumulative distribution curves are generated for each treatment group, and a log-rank test comparing the curves is performed. A corresponding summary table presents by treatment group the cumulative distribution function estimate, the number of participants at risk, the number of participants responding, and the number of participants censored at each post-baseline time point. The table also presents the first quartile, median, and third quartile, along with the corresponding 2-sided 95% CI, of time to response. Time to response for each individual TMA parameter is assessed in a similar manner as time to Complete TMA Response.

The following secondary endpoints are summarized by treatment group by calculating the point estimate and 2-sided 95% CI for the proportion of responders, based on exact confidence limits using the Clopper Pearson method:

(1) Proportion of participants who achieve Hematologic Response at Week 26, as well as each post-baseline time point;
(2) Proportion of participants who achieve Renal Response at Week 26, as well as each post-baseline time point;
(3) Proportion of participants with response in at least 1 TMA parameter at Week 26, as well as each post-baseline time point;
(4) Proportion of participants with an increase of ≥ 2 grams in hemoglobin by Week 26;
(5) Proportion of participants with Complete TMA Response at Week 26 who maintain response at Week 52; and

(6) Among the participants who achieve Complete TMA Response, the proportion of participants who have TMA Relapse.

The following secondary endpoint is summarized by treatment group using descriptive statistics for the observed value as well as the change from baseline: change from baseline in eGFR at Week 26 as well as each post-baseline time point.

For participants who require dialysis, change from baseline in dialysis requirements is summarized. An analysis presents the number and proportion of participants who require dialysis over time. A 2-sided 95% CI for the proportion is provided. A participant is considered as not requiring dialysis at a specific post-baseline time point if they have been dialysis-free for at least 14 days prior to that time point.

All safety analyses are performed on the Safety Set.

Individual PK/PD data is collected for all participants. Graphs of mean serum ravulizumab (ULTOMIRIS®) concentration-time profiles are constructed. Graphs of serum concentration-time profiles for individual participants can also be provided. Descriptive statistics can be calculated for serum concentration data at each sampling time, as appropriate. The PD effects of ravulizumab (ULTOMIRIS®) can be evaluated by assessing the absolute values and changes and percentage changes from Baseline in serum free C5 concentrations over time, as appropriate. Descriptive statistics are calculated for the PD data at each sampling time, as appropriate.

The incidence of ADAs to ravulizumab (ULTOMIRIS®) is presented at each postbaseline time point in tabular format. Additionally, any confirmed ADA-positive samples are tested for titer and the presence of neutralizing antibodies to ravulizumab (ULTOMIRIS®) .

The following quality of life assessments are summarized by treatment group at baseline and each postbaseline time point using descriptive statistics for the observed value as well as the change from baseline: (1) EQ-5D-5L, (2) FACIT-Fatigue, and (3) KDQOL-36.

The primary and key secondary efficacy analyses use a hierarchical stepdown closed-testing procedure. If the null hypothesis for the primary efficacy endpoint is rejected, the key secondary efficacy endpoints are tested in the following order until a non-significant test is observed, at which point no further testing of subsequent endpoints will occur: (1) Time to Complete TMA Response, (2) Hematologic response at Week 26, (3) Change from baseline in eGFR at Week 26, and (4) Proportion of participants on dialysis at Week 26.

An interim analysis to assess sample size re-estimation is performed when 50% of the planned participants (n = 100) have been assessed for the primary endpoint. This interim analysis is performed by an unblinded independent statistical center that is not involved with study conduct or final analysis of study data. Enrollment of participants proceeds without interruption while the analysis is ongoing. There are no plans to stop the study for demonstration of efficacy or futility at the interim analysis.

The sample size re-estimation analysis is based on the conditional power calculated using the results obtained at this interim analysis. If the conditional power falls within the promising zone based on the estimated treatment effect, the sample size is increased up to a maximum of 150 participants. Because the total sample size could potentially be increased in a data-dependent manner following the interim analysis, the final primary analysis for the primary endpoint is tested using the Cui, Hung, Wang method for controlling the type 1 error (Cui, 1999).

The primary efficacy analysis is performed at the end of the 26-Week Treatment Period after all participants have completed or withdrawn from the 26-Week Treatment Period. This analysis allows for evaluation of the primary endpoint.

The final study analysis is conducted at the end of the study.

**K. Clinical Laboratory Test**

The tests detailed below in Table 5 are performed by a central laboratory or designated ancillary laboratories. Additional tests may be performed at any time during the study as determined necessary by the Investigator or required by local regulations.

**Table 5: Protocol-Required Laboratory Assessments**

| Laboratory Assessments | Parameters |
| --- | --- |
| Hematology | • Platelet count<br>• Immature platelet fraction<br>• Hemoglobin (including free hemoglobin)<br>• Hematocrit |

(continued)

| Laboratory Assessments | Parameters |
|---|---|
| | • Red blood cell count<br>• RBC indices<br>◦ Mean corpuscular volume<br>◦ Mean corpuscular hemoglobin<br>◦ Percentage of reticulocytes<br>• White blood cell count with differential (including early progenitors):<br>◦ Neutrophils, segmented<br>◦ Lymphocytes<br>◦ Monocytes<br>◦ Eosinophils<br>◦ Basophils<br>• Haptoglobin<br>• RBC morphology (peripheral blood smear); particularly the presence of schistocytes |
| Coagulation panel | • INR<br>• PT<br>• APTT<br>• D-dimer<br>• Fibrinogen |
| Clinical chemistry | • Lactate dehydrogenase<br>• Liver function tests:<br>    ◦ ALT<br>    ◦ AST<br>    ◦ ALP<br>    ◦ Albumin<br>    ◦ Total protein<br>    ◦ Total bilirubin (direct and indirect)<br>    ◦ GGT<br>• Glucose (nonfasting)<br>• C-reactive protein<br>• Renal function:<br>    ◦ Blood urea nitrogen<br>    ◦ Calcium<br>    ◦ Chloride<br>    ◦ Creatinine |
| |     ◦ Magnesium<br>    ◦ Phosphate<br>    ◦ Potassium<br>    ◦ Sodium<br>    ◦ Total carbon dioxide<br>    ◦ Urea |
| Spot urine studies | • Protein<br>• Creatinine |
| Clinical complement tests | Serum samples will include, but are not limited to, assessments of the following:<br>    • C3 and C4 |

(continued)

| Laboratory Assessments | Parameters |
|---|---|
| PK/PD | • Serum PK<br>• Serum PD (free and total C5) |
| Immunogenicity | • ADA |
| Biomarkers | Exploratory blood samples may include, but are not limited to, assessments of the following:<br>    • Vascular inflammation (eg, sTNF-RI)<br>    • Endothelial damage and/or activation (eg, thrombomodulin and sVCAM-1)<br>    • Complement pathway dysregulation (eg, sC5b-9, C5a, and factor Ba)<br>Exploratory urine biomarkers may include, but are not limited to, assessments of the following:<br>    • Complement pathway dysregulation (eg, sC5b-9 and factor Ba)<br>    • Renal injury biomarkers (eg, neutrophil gelatinase-associated lipocalin [NGAL])<br>Freshly collected whole blood $K_2$EDTA, plasma, and urine samples for RTCA |
| Other screening tests | • ADAMTS13 activity<br>• ST-HUS screen (eg, Shiga toxin enzyme immunoassay/PCR in stool/stool culture/rectal swab<br>• Coombs test, direct<br>• HIV-1 and HIV-2 antibody<br>• Serum follicle-stimulating hormone and estradiol (as needed in WOCBP only)<br>• Serum or urine human chorionic gonadotropin pregnancy test (as needed for WOCBP)[a] |
| Other study-specific tests | • Blood sample for genetics analysis (optional; additional consent required) |

[a] Serum pregnancy test at Screening and EOS/ED.
Abbreviations: ADA = antidrug antibody(ies); ADAMTS13 = a disintegrin and metalloproteinase with a thrombospondin type 1 motif, member 13; ALP = alkaline phosphatase; ALT = alanine aminotransferase; APTT =activated partial thromboplastin time; AST = aspartate aminotransferase; C3, C4, C5, and C5a = complement components 3, 4, 5, and 5a; ED = early discontinuation; EOS = end of study; GGT = gamma-glutamyltransferase; HIV = human immunodeficiency virus; INR = international normalized ratio; $K_2$EDTA = dipotassium ethylenediaminetetraacetic acid; PCR = polymerase chain reaction; PD = pharmacodynamic; PK = pharmacokinetic; PT = prothrombin time; RBC = red blood cell; RTCA = Real Time Complement Activity; sC5b-9 = soluble terminal complement complex C5b-9 (membrane attack complex); ST-HUS = Shiga toxin-related hemolytic uremic syndrome; sTNF-R1 = shed tumor necrosis factor receptor 1; sVCAM-1 = shed vascular cell adhesion molecule 1; WOCBP = woman of childbearing potential.

## SEQUENCE SUMMARY

| |
|---|
| **SEQ ID NO:1**<br>GYIFSNYWIQ |
| **SEQ ID NO:2**<br>EILPGSGSTEYTENFKD |
| **SEQ ID NO:3** |

(continued)

| YFFGSSPNWYFDV |
| --- |

**SEQ ID NO:4**

GASENIYGALN

**SEQ ID NO:5**

GATNLAD

**SEQ ID NO:6**

QNVLNTPLT

**SEQ ID NO:7**

QVQLVQSGAEVKKPGASVKVSCKASGYIFSNYWIQWVRQAPGQGLEWM
GEILPGSGSTEYTENFKDRVTMTRDTSTSTVYMELSSLRSEDTAVYYCARY
FFGSSPNWYFDVWGQGTLVTVSS

**SEQ ID NO:8**

DIQMTQSPSSLSASVGDRVTITCGASENIYGALNWYQQKPGKAPKLLIYGA
TNLADGVPSRFSGSGSGTDFTLTISSLQPEDFATYYCQNVLNTPLTFGQGTK
VEIK

**SEQ ID NO:9**

ASTKGPSVFPLAPCSRSTSESTAALGCLVKDYFPEPVTVSWNSGALTSGVH
TFPAVLQSSGLYSLSSVVTVPSSNFGTQTYTCNVDHKPSNTKVDKTVERKC
CVECPPCPAPPVAGPSVFLFPPKPKDTLMISRTPEVTCVVVDVSQEDPEVQF
NWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPS
DIAVEWESNGQPENNYKTTPPVLDSDGSFFLYSRLTVDKSRWQEGNVFSCS
VMHEALHNHYTQKSLSLSLGK

**SEQ ID NO:10**

QVQLVQSGAEVKKPGASVKVSCKASGYIFSNYWIQWVRQAPGQGLEWM
GEILPGSGSTEYTENFKDRVTMTRDTSTSTVYMELSSLRSEDTAVYYCAR
YFFGSSPNWYFDVWGQGTLVTVSSASTKGPSVFPLAPCSRSTSESTAALGCL
VKDYFPEPVTVSWNSGALTSGVHTFPAVLQSSGLYSLSSVVTVPSSNFGTQTYT
CNVDHKPSNTKVDKTVERKCCVECPPCPAPPVAGPSVFLFPPKPKDTLMISR
TPEVTCVVVDVSQEDPEVQFNWYVDGVEVHNAKTKPREEQFNSTYRVVSVLT
VLHQDWLNGKEYKCKVSNKGLPSSIEKTISKAKGQPREPQVYTLPPSQEEMT
KNQVSLTCLVKGFYPSDIAVEWESNGQPENNYKTTPPVLDSDGSFFLYSRLTV
DKSRWQEGNVFSCSVMHEALHNHYTQKSLSLSLGK

**SEQ ID NO:11**

DIQMTQSPSSLSASVGDRVTITCGASENIYGALNWYQQKPGKAPKLLIYG
ATNLADGVPSRFSGSGSGTDFTLTISSLQPEDFATYYCQNVLNTPLTFGQ
GTKVEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSPV
TKSFNRGEC

**SEQ ID NO:12**

QVQLVQSGAEVKKPGASVKVSCKASGHIFSNYWIQWVRQAPGQGLEW
MGEILPGSGHTEYTENFKDRVTMTRDTSTSTVYMELSSLRSEDTAVYYC
ARYFFGSSPNWYFDVWGQGTLVTVSS

**SEQ ID NO:13**

(continued)

| |
|---|
| ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KCCVECPPCPAPPVAGPSVFLFPPKPKDTLMISRTPEVTCVVVDVSQEDPE VQFNWYVDGVEVHNAKTKPREEQFNSTYRVVSVLTVLHQDWLNGKEYK CKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGN VFSCSVLHEALHSHYTQKSLSLSLGK |

| |
|---|
| **SEQ ID NO:14** |
| QVQLVQSGAEVKKPGASVKVSCKASGHIFSNYWIQWVRQAPGQGLEWM GEILPGSGHTEYTENFKDRVTMTRDTSTSTVYMELSSLRSEDTAVYYCAR YFFGSSPNWYFDVWGQGTLVTVSS ASTKGPSVFPLAPCSRSTSESTAALGCL VKDYFPEPVTVSWNSGALTSGVHTFPAVLQSSGLYSLSSVVTVPSSNFGTQTYT CNVDHKPSNTKVDKTVERKCCVECPPCPAPPVAGPSVFLFPPKPKDTLMISR TPEVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVLHEALHSHYTQKSLSLSLGK |

| |
|---|
| **SEQ ID NO:15** |
| ASTKGPSVFPLAPCSRSTSESTAALGCLVKDYFPEPVTVSWNSGALTSGVH TFPAVLQSSGLYSLSSVVTVTSSNFGTQTYTCNVDHKPSNTKVDKTVERKC CVECPPCPAPPVAGPSVFLFPPKPKDTLYITREPEVTCVVVDVSHEDPEVQF NWYVDGMEVHNAKTKPREEQFNSTFRVVSVLTVVHQDWLNGKEYKCKV SNKGLPAPIEKTISKTKGQPREPQVYTLPPSREEMTKNQVSLTCLVKGFYP SDIAVEWESNGQPENNYKTTPPMLDSDGSFFLYSKLTVDKSRWQQGNVF SCSVMHEALHNHYTQKSLSLSPGK |

| |
|---|
| **SEQ ID NO:16** |
| QVQLVQSGAEVKKPGASVKVSCKASGYIFSNYWIQWVRQAPGQGLEWM GEILPGSGSTEYTENFKDRVTMTRDTSTSTVYMELSSLRSEDTAVYYCAR YFFGSSPNWYFDVWGQGTLVTVSSASTKGPSVFPLAPCSRSTSESTAALG CLVKDYFPEPVTVSWNSGALTSGVHTFPAVLQSSGLYSLSSVVTVTSSNF GTQTYTCNVDHKPSNTKVDKTVERKCCVECPPCPAPPVAGPSVFLFPPKP KDTLYITREPEVTCVVVDVSHEDPEVQFNWYVDGMEVHNAKTKPREEQ FNSTFRVVSVLTVVHQDWLNGKEYKCKVSNKGLPAPIEKTISKTKGQPRE PQVYTLPPSREEMTKNQVSLTCLVKGFYPSDIAVEWESNGQPENNYKTTP PMLDSDGSFFLYSKLTVDKSRWQQGNVFSCSVMHEALHNHYTQKSLSLS PGK |

| |
|---|
| **SEQ ID NO:17** |
| GASENIYHALN |

| |
|---|
| **SEQ ID NO:18** |
| EILPGSGHTEYTENFKD |

| |
|---|
| **SEQ ID NO:19** |
| GHIFSNYWIQ |

| |
|---|
| **SEQ ID NO:20** |

(continued)

QVQLVQSGAEVKKPGASVKVSCKASGHIFSNYWIQWVRQAPGQGLEW MGEILPGSGHTEYTENFKDRVTMTRDTSTSTVYMELSSLRSEDTAVYYC ARYFFGSSPNWYFDVWGQGTLVTVSSASTKGPSVFPLAPCSRSTSESTAALG CLVKDYFPEPVTVSWNSGALTSGVHTFPAVLQSSGLYSLSSVVTVPSSNFGTQT YTCNVDHKPSNTKVDKTVERKCCVECPPCPAPPVAGPSVFLFPPKPKDTLMIS RTPEVTCVVVDVSQEDPEVQFNWYVDGVEVHNAKTKPREEQFNSTYRVVSVL TVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVMHEALHNHYTQKSLSLSLGK

**SEQ ID NO:21**

SYAIS

**SEQ ID NO:22**

GIGPFFGTANYAQKFQG

**SEQ ID NO:23**

DTPYFDY

**SEQ ID NO:24**

SGDSIPNYYVY

**SEQ ID NO:25**

DDSNRPS

**SEQ ID NO:26**

QSFDSSLNAEV

**SEQ ID NO:27**

QVQLVQSGAEVKKPGSSVKVSCKASGGTFSSYAISVWRQAPGQGLEWMGGIGPF FGTANYAQKFQGRVTITADESTSTAYMELSSLRSEDTAVYYCARDTPYFD YWGQGTLVTVSS

**SEQ ID NO:28**

DIELTQPPSVSVAPGQTARISCSGDSIPNYYVYWYQQKPGQAPVLVIYDDSNRPSG IPERFSGSNSGNTATLTISGTQAEDEADYYCQSFDSSLNAEVFGGGTK LTVL

**SEQ ID NO:29**

NYIS

**SEQ ID NO:30**

IIDPDDSYTEYSPSFQG

**SEQ ID NO:31**

YEYGGFDI

**SEQ ID NO:32**

SGDNIGNSYVH

**SEQ ID NO:33**

KDNDRPS

**SEQ ID NO:34**

GTYDIESYV

**SEQ ID NO:35**

EVQLVQSGAEVKKPGESLKISCKGSGYSFTNYISWVRQMPGKGLEWMGIIDPDDS YTEYSPSFQGQVTI SADKSISTAYLQWSSLKASDTAMYYCARYEYGGFDI WGQGTLVTVSS

(continued)

| | |
|---|---|
| **SEQ ID NO:36** | SYELTQPPSVSVAPGQTARISCSGDNIGNSYVHWYQQKPGQAPVLVIYKDNDRPS GIPERFSGSNSGNT ATLTISGTQAEDEADYYCGTYDIESYVFGGGTKLTV L |
| **SEQ ID NO:37** | SSYYVA |
| **SEQ ID NO:38** | AIYTGSGATYKASWAKG |
| **SEQ ID NO:39** | DGGYDYPTHAMHY |
| **SEQ ID NO:40** | QASQNIGSSLA |
| **SEQ ID NO:41** | GASKTHS |
| **SEQ ID NO:42** | QSTKVGSSYGNH |
| **SEQ ID NO:43** | QVQLVESGGGLVQPGGSLRLSCAASGFTSHSSYYVAWVRQAPGKGLEWVGAIYT GSGATYKASWAKGRFTISKDTSKNQVVLTMTNMDPVDTATYYCASDGGYDYPT HAMHYWGQGTLVTVSS |
| **SEQ ID NO:44** | DVVMTQSPSSLSASVGDRVTITCQASQNIGSSLAWYQQKPGQAPRLLIYGASKTH SGVPSRFSGSGSGTDFTLTISSLQPEDVATYYCQSTKVGSSYGNHFGGGTKVEIK |
| **SEQ ID NO:45** | QVQLVESGGGLVQPGRSLRLSCAASGFTVHSSYYMAWVRQAPGKGLEWVGAIF TGSGAEYKAEWAKGRVTISKDTSKNQVVLTMTNMDPVDTATYYCASDAGYDYP THAMHYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTK VDKKVEPKSCDKTHTCPPCPAPELRRGPKVFLFPPKPKDTLMISRTPEVTCVVVDV SHEDPEVKFNWYVDGVEVHNAKTKPREEQYNSTYRVVSVLTVLHQDWLNGKEY KCKVSNKGLPSSIEKTISKAKGQPREPQVYTLPPSREEMTKNQVSLTCLVKGFYPS DIAVEWESNGQPENNYKTTPPVLDSDGSFFLYSKLTVDKSRWQQGNVFSCSVLHE ALHAHYTRKELSLSP |
| **SEQ ID NO:46** | DIQMTQSPSSLSASVGDRVTITCRASQGISSSLAWYQQKPGKAPKLLIYGASETES GVPSRFSGSGSGTDFTLTISSLQPEDFATYYCQNTKVGSSYGNTFGGGTKVEIKRT VAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| **SEQ ID NO:47** | QVQLQESGPGLVKPSETLSLTCTVSGDSVSSSYWTWIRQPPGKGLEWIGYIYYSGS SNYNPSLKSRATISVDTSKNQFSLKLSSVTAADTAVYYCAREGNVDTTMIFDYWG QGTLVTVSS |
| **SEQ ID NO:48** | AIQMTQSPSSLSASVGDRVTITCRASQGIRNDLGWYQQKPGKAPKLLIYAASSLQS GVPSRFAGRGSGTDFTLTISSLQPEDFATYYCLQDFNYPWTFGQGTKVEIK |

(continued)

| SEQ ID NO:49 |
| --- |
| QVQLQESGPGLVKPSETLSLTCTVSGDSVSSSYWTWIRQPPGKGLEWIGYIYYSGS SNYNPSLKSRATISVDTSKNQFSLKLSSVTAADTAVYYCAREGNVDTTMIFDYWG QGTLVTVSSASTKGPSVFPLAPCSRSTSESTAALGCLVKDYFPEPVTVSWNSGALT SGVHTFPAVLQSSGLYSLSSVVTVPSSSLGTKTYTCNVDHKPSNTKVDKRVESKY GPPCPPCPAPEFLGGPSVFLFPPKPKDTLMISRTPEVTCVVVDVSQEDPEVQFNWY VDGVEVHNAKTKPREEQFNSTYRVVSVLTVLHQDWLNGKEYKCKVSNKGLPSSI EKTISKAKGQPREPQVYTLPPSQEEMTKNQVSLTCLVKGFYPSDIAVEWESNGQP ENNYKTTPPVLDSDGSFFLYSRLTVDKSRWQEGNVFSCSVMHEALHNHYTQKSL SLSLGK |
| SEQ ID NO:50 |
| AIQMTQSPSSLSASVGDRVTITCRASQGIRNDLGWYQQKPGKAPKLLIYAASSLQS GVPSRFAGRGSGTDFTLTISSLQPEDFATYYCLQDFNYPWTFGQGTKVEIKRTVA APSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC |

**Aspects of the Invention**

1. A method of treating a human patient with complement-mediated TMA (CM-TMA), particularly CM-TMA with a trigger, the method comprising administering to the patient an effective amount of an anti-C5 antibody, or antigen binding fragment thereof,
wherein the anti-C5 antibody, or antigen binding fragment thereof, comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively.

2. The method of aspect 1, wherein the antibody comprises a variant human Fc region that binds to human neonatal Fc receptor (FcRn), wherein the variant human Fc CH3 region comprises Met-429-Leu and Asn-435-Ser substitutions at residues corresponding to methionine 428 and asparagine 434 of a native human IgG Fc region, each in EU numbering.

3. The method of aspect 1 or 2, wherein the anti-C5 antibody, or antigen binding fragment thereof, comprises a heavy chain variable region as set forth in SEQ ID NO:12 and a light chain variable region as set forth in SEQ ID NO:8.

4. The method of any one of aspects 1-3, wherein the anti-C5 antibody, or antigen-binding fragment thereof, further comprises a heavy chain constant region depicted in SEQ ID NO:13.

5. The method of any one of aspects 1-4, wherein the anti-C5 antibody, or antigen binding fragment thereof, comprises a heavy chain as set forth in SEQ ID NO:14 and a light chain as set forth in SEQ ID NO:11.

6. The method of any one of aspects 1-5, wherein the anti-C5 antibody, or antigen-binding fragment thereof, binds to human C5 at pH 7.4 and 25°C with an affinity dissociation constant $(K_D)$ that is in the range $0.1\ nM \leq K_D \leq 1\ nM$.

7. The method of any one of aspects 1-5, wherein the anti-C5 antibody, or antigen-binding fragment thereof binds to human C5 at pH 6.0 and 25°C with a $K_D \geq 10\ nM$.

8. The method of any one of the preceding aspects, wherein the anti-C5 antibody, or antigen binding fragment thereof, is formulated for intravenous administration.

9. The method of any one of the preceding aspects, wherein the anti-C5 antibody, or antigen binding fragment thereof, is administered to the patient:

(a) once on Day 1 at a dose of: 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg; and
(b) on Day 15 and every eight weeks thereafter at a dose of 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

10. A method of treating a human patient with complement-mediated TMA (CM-TMA), the method comprising administering to the patient an effective amount of an anti-C5 antibody, or antigen binding fragment thereof,

wherein the anti-C5 antibody, or antigen binding fragment thereof, comprises CDR1, CDR2 and CDR3 heavy chain sequences as set forth in SEQ ID NOs: 19, 18 and 3, respectively, and CDR1, CDR2 and CDR3 light chain sequences as set forth in SEQ ID NOs:4, 5 and 6, respectively, and a variant human Fc region that binds to human neonatal Fc receptor (FcRn), wherein the variant human Fc CH3 region comprises Met-429-Leu and Asn-435-Ser substitutions at residues corresponding to methionine 428 and asparagine 434 of a native human IgG Fc region, each in EU numbering, and
wherein the anti-C5 antibody, or antigen binding fragment thereof, is administered to the patient:

(a) once on Day 1 at a dose of: 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg; and
(b) on Day 15 and every eight weeks thereafter at a dose of 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

11. The method of any one of the preceding aspects, wherein the anti-C5 antibody, or antigen binding fragment thereof, is administered to a patient weighing ≥ 30 to < 40 kg:

(a) once on Day 1 at a dose of 1200 mg; and
(b) on Day 15 and every eight weeks thereafter at a dose of
2700 mg.

12. The method of any one of aspects 1-10, wherein the anti-C5 antibody, or antigen binding fragment thereof, is administered to a patient weighing ≥ 40 to < 60 kg:

(a) once on Day 1 at a dose of 2400 mg; and
(b) on Day 15 and every eight weeks thereafter at a dose of
3000 mg.

13. The method of any one of aspects 1-10, wherein the anti-C5 antibody, or antigen binding fragment thereof, is administered to a patient weighing ≥ 60 to < 100 kg:

(a) once on Day 1 at a dose of 2700 mg; and
(b) on Day 15 and every eight weeks thereafter at a dose of
3300 mg.

14. The method of any one of aspects 1-10, wherein the anti-C5 antibody, or antigen binding fragment thereof, is administered to a patient weighing ≥ 100 kg:

(a) once on Day 1 at a dose of 3000 mg; and
(b) on Day 15 and every eight weeks thereafter at a dose of
3600 mg.

15. The method of any one of the preceding aspects, wherein the treatment maintains a serum trough concentration of the anti-C5 antibody of 100 μg/ml or greater during the treatment.

16. The method of any one of the preceding aspects, wherein the treatment maintains a serum trough concentration of the anti-C5 antibody of 200 μg/ml or greater during the treatment.

17. The method of any one of the preceding aspects, wherein the treatment reduces free C5 concentration by greater than 99% throughout the treatment period.

18. The method of any one of the preceding aspects, wherein the anti-C5 antibody, or antigen binding fragment thereof, is administered at a dose of 2700 mg, 3000 mg, 3300 mg, or 3600 mg every eight weeks for up to two years.

19. The method of any one of the preceding aspects, wherein the treatment results in terminal complement inhibition.

20. The method of any one of the preceding aspects, wherein the treatment results in a normalization of platelet count without transfusion support and normalization of LDH levels.

21. The method of any one of the preceding aspects, wherein the treatment results in an improvement of eGFR of $\geq$ 30% compared to baseline.

22. The method of any one of the preceding aspects, wherein the treatment results in a normalization of platelet count without transfusion support, normalization of LDH levels, and an improvement of eGFR of $\geq$ 30% compared to baseline.

23. The method of any one of the preceding aspects, wherein the treatment results in a complete TMA response.

24. The method of any one of the preceding aspects, wherein the treatment produces a shift toward normal levels of one or more biomarkers selected from the group consisting of sTNF-RI, thrombomodulin, sVCAM-1, sC5b-9, C5a, factor Ba, and/or neutrophil gelatinase-associated lipocalin (NGAL).

25. The method of any one of the preceding aspects, wherein the treatment produces a change from baseline in quality of life, as assessed via the Functional Assessment of Chronic Illness Therapy (FACIT)-Fatigue Scale, the EuroQol 5-Dimension 5-Level (EQ-5D-5L) Scale, or the Kidney Disease Quality of Life instrument (KDQOL-36) Scale.

26. The method of any one of the preceding aspects, wherein the treatment prolongs survival of the patient.

27. The method of any one of the preceding aspects, wherein the CM-TMA is associated with a trigger.

28. The method of any one of the preceding aspects, wherein the trigger is autoimmune, an infection, a transplant, one or more drugs, or malignant hypertension.

29. The method of aspect 28, wherein the autoimmune trigger is selected from the group consisting of acquired aplastic anemia, acute disseminated encephalomyelitis (ADEM), acute hemorrhagic leukoencephalitis (ABLE) / Hurst's disease, gammaglobulinemia, (primary), alopecia areata, ankylosing spondylitis (AS), anti-NMDA receptor encephalitis, antiphospholipid syndrome (APS), arteriosclerosis, autism spectrum disorders (ASD), autoimmune Addison's disease (AAD), autoimmune dysautonomia / Autoimmune autonomic ganglionopathy (AAG), autoimmune encephalitis, autoimmune gastritis, autoimmune hemolytic anemia (AIHA), autoimmune hepatitis (AIH), autoimmune hyperlipidemia, autoimmune hypophysitis / lymphocytic hypophysitis, autoimmune inner ear disease (AIED), autoimmune lymphoproliferative syndrome (ALPS), autoimmune myocarditis, autoimmune oophoritis, autoimmune orchitis, autoimmune pancreatitis (AIP) / Immunoglobulin G4-Related Disease (IgG4-RD), autoimmune polyglandular syndromes (Types I, II, & III), autoimmune progesterone dermatitis, autoimmune sudden sensorineural hearing loss (SNHL), balo disease, behçet's disease, birdshot chorioretinopathy / birdshot uveitis, bullous pemphigoid, castleman disease, celiac disease, chagas disease, chronic fatigue syndrome (CFS) / myalgic encephalomyelitis (ME), chronic inflammatory demyelinating polyneuropathy (CIDP), chronic Lyme disease / post-treatment Lyme disease syndrome (PTLDS), chronic urticaria (CU), churg-Strauss syndrome / eosinophilic granulomatosis with polyangiitis (EGPA), cicatricial pemphigoid, cogan's syndrome, cold agglutinin disease, CREST syndrome / limited cutaneous systemic sclerosis, crohn's disease (CD), cronkhite-Canada syndrome (CSS), cryptogenic organizing pneumonia (COP), dermatitis herpetiformis, dermatomyositis, type 1 diabetes, discoid lupus, eressler's syndrome / postmyocardial infarction / postpericardiotomy syndrome, endometriosis, eosinophilic fasciitis, erythema nodosum, essential mixed cryoglobulinemia, evans syndrome, fibrosing alveolitis, giant cell arteritis / temporal arteritis / Horton's disease,glomerulonephritis, goodpasture's syndrome / anti-GBM/anti-TBM disease, granulomatosis with polyangiitis (GPA) / Wegener's granulomatosis, grave's disease, guillain-barré syndrome (GBS), hashimoto's thyroiditis / chronic lymphocytic thyroiditis / autoimmune thyroiditis, henoch-Schönlein purpura / IgA vasculitis, herpes gestationis / pemphigoid gestationis, hypogammaglobulinemia, IgA nephropathy / Berger's disease, immune thrombocytopenia (ITP) / autoimmune thrombocytopenic purpura, interstitial cystitis, juvenile idiopathic arthritis, kawasaki disease, lambert-eaton myasthenic syndrome (LEMS), leukocytoclastic vasculitis, lichen planus, lichen sclerosus, ligneous conjunctivitis, linear IgA disease (LAD) / linear IgA bullous dermatosis (LABD), lupus nephritis, ménière's disease, microscopic polyangiitis (MPA), mixed connective tissue disease (MCTD), mooren's ulcer, mucha-Habermann disease, multiple sclerosis (MS), myasthenia gravis (MG), neuromyelitis optica (NMO) / Devic's disease, narcolepsy, non-length-dependent small fiber sensory neuropathy (SFSN), ocular cicatricial pemphigoid, opsoclonus-myoclonus syndrome (OMS), palindromic rheumatism, palmoplantar pustulosis (type of psoriasis), paraneoplastic cerebellar degeneration, paraneoplastic pemphigus, paroxysmal nocturnal hemoglobinuria (PNH), peripheral uveitis / pars planitis, parsonage turner syndrome, pemphigus foliaceus, pemphigus vulgaris, pernicious anemia, POEMS syndrome, polyarteritis nodosa, polymyalgia rheumatica, polymyositis, postural orthostatic tachychardia syndrome (POTS), primary biliary cholangitis (PBC) / primary biliary cirrhosis, primary sclerosing cholangitis (PSC), psoriasis, psoriatic arthritis, pulmonary fibrosis, idiopathic (IPF), pure red cell aplasia, pyoderma gangrenosum, raynaud's phenomenon, reactive arthritis / Reiter's syndrome, reflex sympathetic dystrophy syndrome, relapsing polychondritis, leg syndrome / Willis-Ekbom disease, rheumatic fever, rheumatoid arthritis, sarcoidosis, schmidt syndrome / autoimmune polyendocrine syndrome type II, systemic sclerosis, scleritis, scleroderma, serpiginous choroidopathy, sjögren's syndrome, stiff person syndrome (SPS), systemic lupus erythematosus (SLE), subacute bacterial endocarditis (SBE), sydenham's chorea, sympathetic ophthalmia, takayasu's arteritis (vasculitis), testicular autoimmunity (vasculitis / orchitis), tolosa-Hunt syndrome, transverse myelitis (TM), tubulointerstitial nephritis uveitis syndrome (TINU), ulcerative colitis (UC), undifferentiated connective tissue disease (UCTD), uveitis (anterior), uveitis (intermediate), uveitis (posterior), vasculitis, vitiligo, and vogt-Koyanagi-Harada syndrome (VKH), particularly wherein the autoimmune condition is not due to cellular or antibody-mediated rejection (AMR).

30. The method of aspect 29, wherein the autoimmune trigger is lupus nephritis or systemic sclerosis.

31. The method of aspect 28, wherein the trigger is an infection selected from the group consisting of a bacterial infection, viral infection, fungal infection, or parasitic infection, with the proviso that the infection is not due to Shiga toxin-producing *Escherichia coli, e.g.,* Shiga toxin-related hemolytic uremic syndrome.

32. The method of aspect 27 or 28, wherein the trigger is a solid organ transplant or a bone marrow transplant, with the proviso that the trigger is not due to hematopoietic stem cell transplant (HSCT).

33. The method of aspect 32, wherein the wherein the trigger is a solid organ transplant selected from the group consisting of a kidney, pancreas, liver, heart, and small bowel transplant.

34. The method of any one of the preceding aspects, further comprising administering best supportive care.

35. The method of aspect 34, wherein the best supportive care is selected from the group consisting of one or more of (a) transfusion support, (b) anti-infectives (e.g., antibiotics, antivirals, and antifungals), (c) renal replacement therapy (dialysis), (d) antihypertensive medications, (e) therapy for TMA associated with lupus nephritis or SSc-TMA, and (f) withdrawal or dose adjustment of the suspected agent for drug-induced TMA.

36. The method of any one of the preceding aspects, wherein the patient with CM-TMA is not

(a) a patient with aHUS, including postpartum aHUS, or any known gene mutation that causes aHUS;
(b) a patient with chronic kidney disease (CKD);
(c) a patient who has developed TMA due to hematopoietic stem cell transplantation (HSCT-TMA);
(d) a patient with a history of primary and secondary glomerular diseases other than lupus;
(e) a patient with primary antiphospholipid antibody syndrome (APS);
(f) a patient with history of Shiga toxin-producing *Escherichia coli* infection, *e.g.,* Shiga toxin-related hemolytic uremic syndrome (STEC-HUS);
(g) a patient with familial or acquired ADAMTS13 (a disintegrin and metalloproteinase with a thrombospondin type 1 motif, member 13) deficiency, *e.g.*, wherein the ADAMTS13 deficiency is attributed to ADAMTS13 activity of less than 5%;
(h) a patient who is positive on a direct Coombs test;
(i) a patient whose kidney biopsy is positive for interstitial fibrosis tubular atrophy, glomerulosclerosis, or crescent formation of at least 50%;
(j) a transplant patient with evidence of cellular or antibody-mediated graft rejection (AMR); or
(k) a patient with any combination of features provided under (a)-(j).

37. A kit for treating complement-mediated TMA (CM-TMA) in a human patient, the kit comprising:

(a) a dose of an anti-C5 antibody, or antigen binding fragment thereof, wherein the anti-C5 antibody, or antigen binding fragment thereof, comprises CDR1, CDR2 and CDR3 heavy chain sequences as set forth in SEQ ID NOs: 19, 18 and 3, respectively, and CDR1, CDR2 and CDR3 light chain sequences as set forth in SEQ ID NOs:4, 5 and 6, respectively; and
(b) instructions for using the anti-C5 antibody, or antigen binding fragment thereof, in the method of any one of aspects 1-34.

38. A kit for treating complement-mediated TMA (CM-TMA) in a human patient, the kit comprising:

(a) a dose of an anti-C5 antibody, or antigen binding fragment thereof, wherein the anti-C5 antibody, or antigen binding fragment thereof, comprises CDR1, CDR2 and CDR3 heavy chain sequences as set forth in SEQ ID NOs: 19, 18 and 3, respectively, and CDR1, CDR2 and CDR3 light chain sequences as set forth in SEQ ID NOs:4, 5 and 6, respectively, and a variant human Fc region that binds to human neonatal Fc receptor (FcRn), wherein the variant human Fc CH3 region comprises Met-429-Leu and Asn-435-Ser substitutions at residues corresponding to methionine 428 and asparagine 434 of a native human IgG Fc region, each in EU numbering; and
(b) instructions for using the anti-C5 antibody, or antigen binding fragment thereof, in the method of any one of aspects 1-34.

39. The kit of aspect 37 or 38, wherein the CM-TMA is associated with a trigger.

40. The kit of aspect 39, wherein the trigger is autoimmune, an infection, a transplant, one or more drugs, or malignant hypertension, with the proviso that the trigger due to autoimmune condition is not due to cellular or antibody mediated rejection (AMR); the trigger due to infection is not due to Shiga toxin-producing *Escherichia coli* infection, *e.g.*, Shiga toxin-related hemolytic uremic syndrome; and the trigger due to transplantation is not due to hematopoietic stem cell transplant (HSCT).

## Claims

1. An anti-C5 antibody, or antigen binding fragment thereof, for use in a method of treating a human patient with complement-mediated TMA (CM-TMA), wherein the anti-C5 antibody, or antigen binding fragment thereof, comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively.

2. The anti-C5 antibody, or antigen binding fragment thereof, for use according to claim 1, wherein the antibody comprises a variant human Fc region that binds to human neonatal Fc receptor (FcRn), wherein the variant human Fc CH3 region comprises Met-429-Leu and Asn-435-Ser substitutions at residues corresponding to methionine 428 and asparagine 434 of a native human IgG Fc region, each in EU numbering.

3. The anti-C5 antibody, or antigen binding fragment thereof, for use according to claim 1 or 2, wherein the anti-C5 antibody, or antigen binding fragment thereof:

(a) comprises a heavy chain variable region as set forth in SEQ ID NO:12 and a light chain variable region as set forth in SEQ ID NO:8;
(b) further comprises a heavy chain constant region depicted in SEQ ID NO:13; and/or
(c) comprises a heavy chain as set forth in SEQ ID NO:14 and a light chain as set forth in SEQ ID NO:11.

4. The anti-C5 antibody, or antigen binding fragment thereof, for use according to any one of claims 1 to 3, wherein the anti-C5 antibody, or antigen-binding fragment thereof:

(a) binds to human C5 at pH 7.4 and 25°C with an affinity dissociation constant ($K_D$) that is in the range 0.1 nM $\leq K_D \leq$ 1 nM; or
(b) binds to human C5 at pH 6.0 and 25°C with a $K_D \geq$ 10 nM.

5. The anti-C5 antibody, or antigen binding fragment thereof, for use according to any one of the preceding claims, wherein the anti-C5 antibody, or antigen binding fragment thereof, is formulated for intravenous administration.

6. The anti-C5 antibody, or antigen binding fragment thereof, for use according to any one of the preceding claims, wherein the anti-C5 antibody, or antigen binding fragment thereof, is administered to the patient:

(a) once on Day 1 at a dose of: 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg; and
(b) on Day 15 and every eight weeks thereafter at a dose of 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

7. The anti-C5 antibody, or antigen binding fragment thereof, for use according to any one of the preceding claims, wherein the anti-C5 antibody, or antigen binding fragment thereof, is administered to:

(i) a patient weighing ≥ 30 to < 40 kg:

(a) once on Day 1 at a dose of 1200 mg; and
(b) on Day 15 and every eight weeks thereafter at a dose of 2700 mg;

(ii) a patient weighing ≥ 40 to < 60 kg:

(a) once on Day 1 at a dose of 2400 mg; and
(b) on Day 15 and every eight weeks thereafter at a dose of 3000 mg;

(iii) a patient weighing ≥ 60 to < 100 kg:

(a) once on Day 1 at a dose of 2700 mg; and
(b) on Day 15 and every eight weeks thereafter at a dose of 3300 mg; or

(iv) a patient weighing ≥ 100 kg:

(a) once on Day 1 at a dose of 3000 mg; and
(b) on Day 15 and every eight weeks thereafter at a dose of 3600 mg.

8. The anti-C5 antibody, or antigen binding fragment thereof, for use according to any one of the preceding claims, wherein the treatment:

(a) maintains a serum trough concentration of the anti-C5 antibody of 100 μg/ml or greater, or 200 μg/ml or greater during the treatment; and/or
(b) reduces free C5 concentration by greater than 99% throughout the treatment period.

9. The anti-C5 antibody, or antigen binding fragment thereof, for use according to any one of the preceding claims, wherein the anti-C5 antibody, or antigen binding fragment thereof, is administered at a dose of 2700 mg, 3000 mg, 3300 mg, or 3600 mg every eight weeks for up to two years.

10. The anti-C5 antibody, or antigen binding fragment thereof, for use according to any one of the preceding claims, wherein the treatment:

(a) results in terminal complement inhibition;
(b) results in a normalization of platelet count without transfusion support and normalization of LDH levels;
(c) results in an improvement of eGFR of ≥ 30% compared to baseline;
(d) results in a normalization of platelet count without transfusion support, normalization of LDH levels, and an improvement of eGFR of ≥ 30% compared to baseline;
(e) results in a complete TMA response;
(f) produces a shift toward normal levels of one or more biomarkers selected from the group consisting of sTNF-RI, thrombomodulin, sVCAM-1, sC5b-9, C5a, factor Ba, and/or neutrophil gelatinase-associated lipocalin (NGAL);
(g) produces a change from baseline in quality of life, as assessed via the Functional Assessment of Chronic Illness Therapy (FACIT)-Fatigue Scale, the EuroQol 5-Dimension 5-Level (EQ-5D-5L) Scale, or the Kidney Disease Quality of Life instrument (KDQOL-36) Scale; and/or

(h) prolongs survival of the patient.

11. The anti-C5 antibody, or antigen binding fragment thereof, for use according to any one of the preceding claims, wherein the CM-TMA is associated with a trigger; optionally wherein the trigger is:

(a) autoimmune, an infection, a transplant, one or more drugs, or malignant hypertension; or
(b) a solid organ transplant or a bone marrow transplant, with the proviso that the trigger is not due to hematopoietic stem cell transplant (HSCT); optionally wherein the solid organ transplant is selected from the group consisting of a kidney, pancreas, liver, heart, and small bowel transplant.

12. The anti-C5 antibody, or antigen binding fragment thereof, for use according to claim 11, wherein the autoimmune trigger:

(a) is selected from the group consisting of acquired aplastic anemia, acute disseminated encephalomyelitis (ADEM), acute hemorrhagic leukoencephalitis (ABLE) / Hurst's disease, gammaglobulinemia, (primary), alopecia areata, ankylosing spondylitis (AS), anti-NMDA receptor encephalitis, antiphospholipid syndrome (APS), arteriosclerosis, autism spectrum disorders (ASD), autoimmune Addison's disease (AAD), autoimmune dysautonomia / Autoimmune autonomic ganglionopathy (AAG), autoimmune encephalitis, autoimmune gastritis, autoimmune hemolytic anemia (AIHA), autoimmune hepatitis (AIH), autoimmune hyperlipidemia, autoimmune hypophysitis / lymphocytic hypophysitis, autoimmune inner ear disease (AIED), autoimmune lymphoproliferative syndrome (ALPS), autoimmune myocarditis, autoimmune oophoritis, autoimmune orchitis, autoimmune pancreatitis (AIP) / Immunoglobulin G4-Related Disease (IgG4-RD), autoimmune polyglandular syndromes (Types I, II, & III), autoimmune progesterone dermatitis, autoimmune sudden sensorineural hearing loss (SNHL), balo disease, behçet's disease, birdshot chorioretinopathy / birdshot uveitis, bullous pemphigoid, castleman disease, celiac disease, chagas disease, chronic fatigue syndrome (CFS) / myalgic encephalomyelitis (ME), chronic inflammatory demyelinating polyneuropathy (CIDP), chronic Lyme disease / post-treatment Lyme disease syndrome (PTLDS), chronic urticaria (CU), churg-Strauss syndrome / eosinophilic granulomatosis with polyangiitis (EGPA), cicatricial pemphigoid, cogan's syndrome, cold agglutinin disease, CREST syndrome / limited cutaneous systemic sclerosis, crohn's disease (CD), cronkhite-Canada syndrome (CSS), cryptogenic organizing pneumonia (COP), dermatitis herpetiformis, dermatomyositis, type 1 diabetes, discoid lupus, eressler's syndrome / postmyocardial infarction / postpericardiotomy syndrome, endometriosis, eosinophilic fasciitis, erythema nodosum, essential mixed cryoglobulinemia, evans syndrome, fibrosing alveolitis, giant cell arteritis / temporal arteritis / Horton's disease, glomerulonephritis, goodpasture's syndrome / anti-GBM/anti-TBM disease, granulomatosis with polyangiitis (GPA) / Wegener's granulomatosis, grave's disease, guillain-barré syndrome (GBS), hashimoto's thyroiditis / chronic lymphocytic thyroiditis / autoimmune thyroiditis, henoch-Schönlein purpura / IgA vasculitis, herpes gestationis / pemphigoid gestationis, hypogammaglobulinemia, IgA nephropathy / Berger's disease, immune thrombocytopenia (ITP) / autoimmune thrombocytopenic purpura, interstitial cystitis, juvenile idiopathic arthritis, kawasaki disease, lambert-eaton myasthenic syndrome (LEMS), leukocytoclastic vasculitis, lichen planus, lichen sclerosus, ligneous conjunctivitis, linear IgA disease (LAD) / linear IgA bullous dermatosis (LABD), lupus nephritis, ménière's disease, microscopic polyangiitis (MPA), mixed connective tissue disease (MCTD), mooren's ulcer, mucha-Habermann disease, multiple sclerosis (MS), myasthenia gravis (MG), neuromyelitis optica (NMO) / Devic's disease, narcolepsy, non-length-dependent small fiber sensory neuropathy (SFSN), ocular cicatricial pemphigoid, opsoclonus-myoclonus syndrome (OMS), palindromic rheumatism, palmoplantar pustulosis (type of psoriasis), paraneoplastic cerebellar degeneration, paraneoplastic pemphigus, paroxysmal nocturnal hemoglobinuria (PNH), peripheral uveitis / pars planitis, parsonage turner syndrome, pemphigus foliaceus, pemphigus vulgaris, pernicious anemia, POEMS syndrome, polyarteritis nodosa, polymyalgia rheumatica, polymyositis, postural orthostatic tachychardia syndrome (POTS), primary biliary cholangitis (PBC) / primary biliary cirrhosis, primary sclerosing cholangitis (PSC), psoriasis, psoriatic arthritis, pulmonary fibrosis, idiopathic (IPF), pure red cell aplasia, pyoderma gangrenosum, raynaud's phenomenon, reactive arthritis / Reiter's syndrome, reflex sympathetic dystrophy syndrome, relapsing polychondritis, leg syndrome / Willis-Ekbom disease, rheumatic fever, rheumatoid arthritis, sarcoidosis, schmidt syndrome / autoimmune polyendocrine syndrome type II, systemic sclerosis, scleritis, scleroderma, serpiginous choroidopathy, sjögren's syndrome, stiff person syndrome (SPS), systemic lupus erythematosus (SLE), subacute bacterial endocarditis (SBE), sydenham's chorea, sympathetic ophthalmia, takayasu's arteritis (vasculitis), testicular autoimmunity (vasculitis / orchitis), tolosa-Hunt syndrome, transverse myelitis (TM), tubulointerstitial nephritis uveitis syndrome (TINU), ulcerative colitis (UC), undifferentiated connective tissue disease (UCTD), uveitis (anterior), uveitis (intermediate), uveitis (posterior), vasculitis, vitiligo, and vogt-Koyanagi-Harada syndrome (VKH), particularly wherein the autoimmune condition is not due to cellular or antibody-mediated rejection (AMR); or
(b) is lupus nephritis or systemic sclerosis.

13. The anti-C5 antibody, or antigen binding fragment thereof, for use according to claim 11, wherein the trigger is an infection selected from the group consisting of a bacterial infection, viral infection, fungal infection, or parasitic infection, with the proviso that the infection is not due to Shiga toxin-producing *Escherichia coli, e.g.,* Shiga toxin-related hemolytic uremic syndrome.

14. The anti-C5 antibody, or antigen binding fragment thereof, for use according to any one of the preceding claims, further comprising administering best supportive care, optionally wherein the best supportive care is selected from the group consisting of one or more of (a) transfusion support, (b) anti-infectives (*e.g.*, antibiotics, antivirals, and antifungals), (c) renal replacement therapy (dialysis), (d) antihypertensive medications, (e) therapy for TMA associated with lupus nephritis or SSc-TMA, and (f) withdrawal or dose adjustment of the suspected agent for drug-induced TMA.

15. The anti-C5 antibody, or antigen binding fragment thereof, for use according to any one of the preceding claims, wherein the patient with CM-TMA is not:

   (a) a patient with aHUS, including postpartum aHUS, or any known gene mutation that causes aHUS;
   (b) a patient with chronic kidney disease (CKD);
   (c) a patient who has developed TMA due to hematopoietic stem cell transplantation (HSCT-TMA);
   (d) a patient with a history of primary and secondary glomerular diseases other than lupus;
   (e) a patient with primary antiphospholipid antibody syndrome (APS);
   (f) a patient with history of Shiga toxin-producing *Escherichia coli* infection, *e.g.,* Shiga toxin-related hemolytic uremic syndrome (STEC-HUS);
   (g) a patient with familial or acquired ADAMTS13 (a disintegrin and metalloproteinase with a thrombospondin type 1 motif, member 13) deficiency, *e.g.,* wherein the ADAMTS13 deficiency is attributed to ADAMTS13 activity of less than 5%;
   (h) a patient who is positive on a direct Coombs test;
   (i) a patient whose kidney biopsy is positive for interstitial fibrosis tubular atrophy, glomerulosclerosis, or crescent formation of at least 50%;
   (j) a transplant patient with evidence of cellular or antibody-mediated graft rejection (AMR); or
   (k) a patient with any combination of features provided under (a)-(j).

| Screening Period (≤ 14 days) | Treatment Period (26 weeks) | Post-treatment Follow-up Period (26 weeks) |

N = ~100

Randomization 1:1

Ravulizumab + BSC
N = 50

N = 50
Placebo + BSC

Day 1

Week 26
Primary Analysis

Week 52
End of Study

Note: Randomized participants receive a weight-based loading dose on Day 1, followed by weight-based maintenance dosing on Day 15 and then q8w. Weight-based dosing regimen is based on last recorded study visit body weight. Abbreviations: BSC = best supportive care; q8w = every 8 weeks; TMA = thrombotic microangiopathy.

**FIG. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63140488
- US 8241628 B
- US 8883158 B
- US 20160176954 A1
- US 20170355757 A
- US 1995005688 W

- US 6355245 B
- US 2007006606 W
- US 2015019225 W
- US 9079949 B
- US 8088376 B


**Non-patent literature cited in the description**

- **AIGNER C et al.** *Clin. Kidney J.,* 2019, vol. 12 (3), 333-337
- **GO RS et al.** *Mayo Clin. Proc.,* 2016, vol. 91 (9), 1189-1211
- **GOODSHIP THJ et al.** *Kidney Int.,* 2017, vol. 91 (3), 539-551
- **PARK et al.** *Blood Advances,* 2018, vol. 2 (16), 2090-2094
- **LE CLECH A et al.** *Kidney Intl.,* 2019, vol. 95 (6), 1443-1452
- **AZOULAY E et al.** *Chest,* 2017, vol. 152 (2), 424-434
- **KAVANAGH D et al.** *Br. Med. Bull.,* 2006, vol. 77-78 (5-22), 5-22
- **LAURENCE.** *Clin. Adv. Hematol. Oncol.,* 2013, vol. 11 (15), 4-15
- **FUKUZAWA T. et al.** *Sci. Rep.,* 2017, vol. 7, 1080
- Sequences of Proteins of Immunological Interest. **KABAT et al.** NIH Publication. U.S. Department of Health and Human Services, 1991
- **CHOTHIA et al.** *Nature,* 1989, vol. 342, 877-83
- **THOMAS et al.** *Mol. Immunol.,* 1996, vol. 33, 1389-401
- **DALL'ACQUA, W et al.** *J. Biol. Chem.,* 2006, vol. 281, 23514-24
- **HINTON, P. et al.** *J. Biol. Chem.,* 2004, vol. 279, 6213-6
- **HINTON, P. et al.** *J. Immunol.,* vol. 176, 346-56
- **PETKOVA, S. et al.** *Int. Immunol.,* 2006, vol. 18, 1759-69
- **DATTA-MANNAN, A. et al.** *J. Biol. Chem.,* 2007, vol. 282, 1709-17
- **KÖHLER, G ; MILSTEIN, C.** *Eur. J. Immunol.,* 1976, vol. 6, 511-9
- **HUSE, W et al.** *Science,* 1989, vol. 246, 1275-81

- **GENNARO.** Remington: The Science and Practice of Pharmacy. Lippincott, Williams & Wilkins, 2000
- **ANSEL et al.** Pharmaceutical Dosage Forms and Drug Delivery Systems. Lippincott Williams & Wilkins Publishers, 1999
- **KIBBE.** Handbook of Pharmaceutical Excipients American Pharmaceutical Association. 2000
- **CAPRIOLI et al.** *Blood,* 2006, vol. 108, 1267-1279
- **ESPARZA-GORDILLO et al.** *Hum Mol Genet,* 2005, vol. 14, 703-712
- **KAPLAN et al.** Hemolytic Uremic Syndrome and Thrombotic Thrombocytopenic Purpura. Informa Health Care, 1992
- **ZIPFEL.** Complement and Kidney Disease. Springer, 2005
- **BRESLIN et al.** *Clin Am Soc Nephrol,* 2006, vol. 1, 88-99
- **GOICOECHEA DE JORGE et al.** *Proc Natl Acad Sci USA,* 2007, vol. 104, 240-245
- **LEVEY AS et al.** *Ann Intern Med,* 2009, vol. 150 (9), 604-612
- **TAYLOR et al.** *Thromb Haemost.,* 2001, vol. 86 (5), 1327-13300
- **SINGER M et al.** *JAMA,* 2016, vol. 315 (8), 801-810
- **CAIRES RA et al.** *Transplant Proc.,* 2012, vol. 44 (8), 2388-2390
- **HUMPHREYS BD et al.** *Cancer,* 2004, vol. 100 (12), 2664-2670
- **LEE C-S et al.** *Nephrology,* 2012, vol. 17 (1), 48-52
- **SCHWARZ A et al.** *Am J Transplant.,* 2010, vol. 10 (9), 2017-2025
- **SONG D et al.** *Arthritis Res Ther,* 2013, vol. 15 (1), R12-R12
- **WATERS et al.** *J Pediatr.,* 2007, vol. 151 (2), 140-144

- **WU L-H et al.** *Kidney Int Suppl.,* 2013, vol. 83 (4), 715-723